# EUROPEAN PATENT SPECIFICATION

(11) **EP 2 747 711 B1**
(45) Date of publication and mention of the grant of the patent: **27.02.2019**
(21) Application number: 12825367.1
(22) Date of filing: 22.08.2012
(51) Int. Cl.: A61F 2/28, A61C 19/06, A61C 8/00, A61F 2/30, A61F 2/36, A61L 27/04, A61L 27/06, A61L 27/30, A61L 27/32, A61L 27/50, A61L 27/54, A61L 31/08, A61L 31/16

(54) **MEDICAL DEVICE FOR BONE IMPLANT AND METHOD FOR PRODUCING SUCH A DEVICE**
MEDIZINISCHE VORRICHTUNG FÜR KNOCHENIMPLANTAT UND VERFAHREN ZUR HERSTELLUNG EINER DERARTIGEN VORRICHTUNG
DISPOSITIF MÉDICAL POUR IMPLANT OSSEUX ET PROCÉDÉ DE PRODUCTION DUDIT DISPOSITIF

(30) Priority: 22.08.2011 US 201161526196 P
(43) Date of publication of application: 02.07.2014
(73) Proprietor: Exogenesis Corporation, Billerica, Massachusetts 01821 (US)
(72) Inventor: TARRANT, Laurence, B., Billerica, Massachusetts 01821 (US); SVRLUGA, Richard, C., Billerica, Massachusetts 01821 (US); KIRKPATRICK, Sean, R., Billerica, Massachusetts 01821 (US)
(74) Representative: FRKelly
(86) International application number: PCT/US2012/051816
(87) International publication number: WO 2013/028735

(56) References cited:
- WO-A1-2006/050106
- US-A1- 2007 181 820
- US-A1- 2010 036 502
- US-A1- 2010 036 502
- US-A1- 2011 086 081

## Description

### FIELD OF THE INVENTION

This invention relates generally to an implantable medical device for implantation into or onto a bone of a mammal and to a method for producing such an implantable medical device. More specifically, it relates to an implantable medical device made from a biocompatible material, preferably titanium (with a titania surface) or zirconia, with at least a portion of its surface parts modified to facilitate improved integration with bone. The invention also relates to a method for producing such an implant that includes the use of accelerated Neutral Beam technology, wherein the accelerated Neutral Beam is derived from gas cluster ion beam (GCIB).

### BACKGROUND OF THE INVENTION

As used herein the term "titania" is intended to include oxides of titanium, and the titanium metal itself (or an alloy thereof) together with a surface coating of native oxide or other oxide comprising the element titanium (including without limitation TiO₂, and or TiO₂ with imperfect stoichiometry).

As used herein the term "zirconia" is intended to mean zirconium dioxide (even with imperfect stoichiometry) in any of its various forms (treated or untreated to toughen it for use in a bone implant) and any materials or ceramics that are at least 50% zirconium dioxide.

As used herein, the term "tricalcium phosphate" is intended to include without limitation beta tricalcium phosphate.

As used herein, the term "nutrient material" is intended to include any material that encourages osteoblasts to grow and produce bone components on a surface by providing a local nutrient source. Nutrient material examples include, without limitation, calcium phosphate-containing materials such as hydroxyapatite [Ca₅(PO₄)₃(OH)]ₓ (HA) or tricalcium phosphate Ca₃(PO₄)₂ (TCP); or other minerals or compounds having a composition similar to natural bone components including Bioglass 45S5 and Bioglass 58S; or compounds related to the foregoing but having imperfect stoichiometry; or other sources of Ca, Ca⁺⁺, P, O, PO₄, or P₂O₅; or molecular dissociation products including Ca, P, O, and H atoms, as well as larger fragments of the HA or TCP molecules.

As used herein, the term "BMP" is intended to include any of the bone morphogenic proteins that are useful in promoting the formation and/or attachment of new bone growth when applied in contact with or in proximity to a bone-implantable medical device.

As used herein, the term "bone growth-stimulating agent" is intended to include any material that stimulates and encourages the development and functional maintenance of mature osteoblasts. Bone growth-stimulating agents include, without limitation: growth factors; cytokines and the like, such as members of the Transforming Growth Factor-beta (TGF-β) protein superfamily including any of the Bone Morphogenic Proteins (BMP) and members of Glycosylphosphatidylinositol-anchored (GPI-anchored) signaling proteins including members of the Repulsive Guidance Molecule (RGM) protein family; and other growth regulatory proteins.

As used herein the term "osteoinductive agent" is intended to mean a nutrient material and/or a bone growth-stimulating agent.

As used herein, the term "hole" is intended to mean any hole, cavity, crater, trough, trench, or depression penetrating a surface of a bone-implantable medical device and may extend through a portion of the device (through-hole), or only part way through the device (blind-hole, or cavity) and may be substantially cylindrical, rectangular, or of any other shape.

As used herein, the term "bone-implantable medical device" is intended to include, without limitation, dental implants, bone screws, interference screws, buttons, artificial joint prostheses (as for example femoral ball prostheses or an acetabular cup prostheses) that attach to a bone, and endosseus implants, prostheses and supports or any implant that required the integration of bone with the implant, and ceramic, polymeric, metallic, or hybrid materials that are meant to affix ligaments, tendons, rotator cuffs, and the like soft skeletal tissues to bony tissues.

As used herein, the term "therapeutic agent" is intended to mean a medicine, drug, antibiotic, anti-inflammatory agent, osteoinductive agent, BMP, or other material that is bioactive in a generally beneficial way.

As used herein, the term "elution" is intended to mean the release of an at least somewhat soluble drug material from a drug source on a medical device or in a hole in a medical device by gradual solution of the drug in a solvent, typically a bodily fluid solvent encountered after implantation of the medical device in a subject. In many cases the solubility of a drug material is high enough that the release of the drug into solution occurs more rapidly than desired, undesirably shortening the therapeutic lifetime of the drug following implantation of the medical device. The rate of elution or rate of release of the drug may depend on many factors such as for examples, solubility of the drug or exposed surface area between the drug and the solvent or mixture of the drug with other materials to reduce solubility. However, barrier or encapsulating layers between the drug and solvent can also modify the rate of elution or release of the drug. It is often desirable to delay the rate of release by elution to extend the time of therapeutic influence at the implant site. The desired elution rates are well known per se to those working in the arts of the medical devices. The present invention enhances their control of those rates in the devices. See, e.g. http://www.news-medical.net/health/Drug-Eluting-Stent-Design.aspx (duration of elution). US 3,641,237 teaches some specific drug elution rates. Haery et al., "Drug-eluting stents: The beginning of the end of restenosis?", Cleveland Clinic Journal of Medicine, V71(10), (2004), includes some details of drug release rates for stents at pg. 818, Col. 2, paragraph 5.

As used herein, the term "diffusion" is intended to mean the concentration gradient driven transport of a material across or through a barrier layer. A fluid (such as a biological fluid) diffusing across a barrier layer typically results in a molecular scale movement from the side on which the fluid is more abundant to the side where it is less abundant, with a resulting concentration gradient within the layer.

Bone implantable medical devices intended for implant into or onto the bones of a mammal (including human) are employed as anchors for dental restoration, fasteners and/or prostheses for repair of bone fractures, joint replacements, and other applications requiring attachment to bone. It is known that titania and zirconia are among preferred materials for such bone-implantable medical devices because of the biocompatibility of the material and its ability to accept attachment of new bone growth, however other materials including stainless steel alloys, cobalt-chrome alloy, cobalt-chrome-molybdenum alloy, other ceramics in addition to zirconia, and other materials are also utilized. Bone-implantable medical devices are often fabricated from titanium metal (or alloy) that typically has a titania surface (either native oxide or otherwise). Bone-implantable medical devices may be coated (or partially coated) with (A) one or more nutrient materials or (B) one or more bone growth-stimulating agents. Such materials may be applied as a coating by a variety of techniques. Bone growth-stimulating agents may be introduced into a surgical implantation site or applied as coatings for implantable medical devices and also may serve to facilitate new bone growth and attachment for integration of the device into the bone. Bone growth-stimulating agents may be used as an alternative to or in combination with nutrient material coatings. Coatings of nutrient materials may be partial, and if totally contiguous on the surface, may actually discourage adhesion of the cells and bone integration by leaving exposed gaps on the surface as consumed.

Other problems exist, in that when such medical devices are being implanted into bone, the surfaces of the devices most intimately in contact with the preexisting bone often experience considerable mechanical abrasion and/or wiping by the bone. For example, an anchor for a dental implant often consists of a threaded screw portion that is screwed into a drilled bone hole and, which effectively becomes a self-tapping screw during implant, cutting its own threads in the drilled hole. Similarly, orthopedic bone screws for repairing fractures or attaching prostheses to immobilize fractures, also experience considerable abrading forces on the threaded surfaces during their surgical placement. An artificial hip joint prosthesis has a stem for insertion into a hole in a femur, and may be forcibly hammered into the opening during surgical implantation, undergoing abrading forces on the inserted stem. In such procedures, the aggressive abrasion of the surfaces of the medical devices during their implantation tends to abrade away or otherwise result in premature removal or release of attached osteoinductive agents. This results in reduced benefit from the osteoinductive coatings, which in turn results in longer times for complete integration of the implant into the bone. Longer integration times often correspond to delayed healing and increased costs and greater suffering for the mammal receiving the implant.

Bone-implantable medical devices having holes or grooves for retaining and delivering osteoinductive agents are known. This approach provides relief for some of the problems described above. However, in general, medicines so delivered may not be adequately retained and may migrate or elute out of the holes more rapidly than is desired for optimal effect. One response to this problem has been to mix the medicine with a polymer prior to loading it into the holes. This can result in slowed release of the medicine as the polymer biodegrades and/or erodes. Another response has been to load the medicine and then cover it with a polymer layer. This can result in delayed or slowed release. In either case the intent and effect is to delay and/or control the elution of the medicine from the hole, extending its therapeutic lifetime and effectiveness. There remain a number of problems associated with this polymer technology. Because of the mechanical forces involved in the implantation of a bone-implantable medical device, the polymeric material has a tendency to crack and sometimes delaminate. This modifies the medicine release rate from that which is intended and additionally the polymeric flakes can migrate through the osteosurgical site and cause unintended side effects. There is evidence to suggest that the polymers themselves cause a toxic reaction that may interfere with proper healing and with long-term success. Additionally, because of the volume of polymer required to adequately contain the medicine, the total amount of medicine that can be loaded may be undesirably reduced.

Gas cluster ion beams are generated and transported for purposes of irradiating a workpiece according to known techniques. Various types of holders are known in the art for holding the object in the path of the GCIB for irradiation and for manipulating the object to permit irradiation of a multiplicity of portions of the object. Neutral Beams may be generated and transported for purposes of irradiating a workpiece according to techniques taught herein.

GCIB have been employed to smooth or otherwise modify the surfaces of implantable medical devices such as stents, joint prostheses and other implantable medical devices. For example, U.S. Pat. 6,676,989C1 issued to Kirkpatrick et al. teaches a GCIB processing system having a holder and manipulator suited for processing tubular or cylindrical workpieces. In another example, U.S. Pat. 6,491,800B2 issued to Kirkpatrick et al. teaches a GCIB processing system having workpiece holders and manipulators for processing other types of non-planar medical devices, including for example, hip joint prostheses. In view of the increasing use of surgical implants into or onto bone, the value of the use of osteoinductive agents, and the problems associated with state of the art practice, it is desirable to have bone-implantable medical devices that can be loaded with osteoinductive agents and which are resistant to the forces and abrasions encountered during the implantation process, thus providing superior retention for greater post-implant effectiveness.

Gas cluster ion beams have been successfully used to form HA-infused layers and infused layers of other osteoinductive agents on devices having titanium and titania surfaces intended for implant into bone, and use of GCIB represents a step forward in such technology. However, some problems have not been fully solved by GCIB technology, as will be shown herein and it will be shown that the invention addresses these outstanding problems.

Ions have long been favored for many processes because their electric charge facilitates their manipulation by electrostatic and magnetic fields. This introduces great flexibility in processing. However, in some applications, the charge that is inherent to any ion (including gas cluster ions in a GCIB) may produce undesirable effects in the processed surfaces. GCIB has a distinct advantage over conventional ion beams in that a gas cluster ion with a single or small multiple charge enables the transport and control of a much larger mass-flow (a cluster may consist of hundreds or thousands of molecules) compared to a conventional ion (a single atom, molecule, or molecular fragment.) Particularly in the case of insulating materials, and materials with poor electrical conductivity (as for example some drugs and therapeutic and osteoinductive agents) surfaces processed using ions often suffer from charge-induced damage resulting from abrupt discharge of accumulated charges, or production of damaging electrical field-induced stress in the material (again resulting from accumulated charges.) In many such cases, GCIBs have an advantage due to their relatively low charge per mass, but in some instances may not eliminate the target-charging problem. Furthermore, moderate to high current intensity ion beams may suffer from a significant space charge-induced defocusing of the beam that tends to inhibit transporting a well-focused beam over long distances. Again, due to their lower charge per mass relative to conventional ion beams, GCIBs have an advantage, but they do not fully eliminate the space charge transport problem.

A further instance of need or opportunity arises from the fact that although the use of beams of neutral molecules or atoms provides benefit in some surface processing applications and in space charge-free beam transport, it has not generally been easy and economical to produce intense beams of neutral molecules or atoms except for the case of nozzle jets, where the energies are generally on the order of a few milli-electron-volts per atom or molecule, and thus have limited processing capabilities. More energetic neutral particles can be beneficial or necessary in many applications, for example when it is desirable to break surface or shallow subsurface bonds to facilitate cleaning, etching, smoothing, deposition, amorphization, or to produce surface chemistry effects. In such cases, energies of from about an eV up to a few thousands of eV per particle can often be useful. Methods and apparatus for forming such Neutral Beams by first forming an accelerated charged GCIB and then neutralizing or arranging for neutralization of at least a fraction of the beam and separating the charged and uncharged fractions are disclosed herein. The Neutral Beams may consist of neutral gas clusters, neutral monomers, or a combination of both. Although GCIB processing has been employed successfully for many applications, there are new and existing application needs not fully met by GCIB or other state of the art methods and apparatus, and wherein accelerated Neutral Beams may provide superior results. For example, in many situations, while a GCIB can produce dramatic atomic-scale smoothing of an initially somewhat rough surface, the ultimate smoothing that can be achieved is often less than the required smoothness, and in other situations GCIB processing can result in roughening moderately smooth surfaces rather than smoothing them further.

It is therefore an object of this invention to provide bone-implantable medical devices having surfaces with improved retention of osteoinductive agents.

It is further an objective of this invention to provide methods of attaching and/or retaining osteoinductive agents on surfaces of bone-implantable medical devices.

Yet another objective (which is not part of the present invention) is to provide bone-implantable medical devices and methods for their production that retain medicines or other therapeutic agents in holes with controlled release or elution rates and without the undesirable effects associated with the use of polymers by employing accelerated Neutral Beam technology.

United States Patent specification no US - A - 2010/036502 discloses a bone implantable medical device made from a biocompatible material, preferably comprising titania or zirconia, that has at least a portion of its surface modified to facilitate improved integration with bone. The implantable device may incorporate a surface infused with osteoinductive agent and/or may incorporate holes loaded with a therapeutic agent. The infused surface and/or the holes may be patterned to determine the distribution of and amount of osteoinductive agent and/or therapeutic agent incorporated. The rate of release or elution profile of the therapeutic agent may be controlled.

### SUMMARY OF THE INVENTION

According to an aspect of the present invention, there is provided a method of modifying a surface of a bone-implantable medical device as specified in claim 1. According to another aspect of the present invention, there is provided a method of modifying a surface of a bone-implantable medical device as specified in any of claims 2 - 14. According to yet another aspect of the present invention, there is provided a bone-implantable medical device having a surface as specified in claim 15. According to yet further aspect of the present invention, there is provided a bone-implantable medical device having a surface as specified in claim 16 or claim 17.

The objects set forth above as well as further and other objects and advantages of the present invention are achieved by the invention described herein below.

The present invention is directed to the use of accelerated Neutral Beam processing to form one or more surface regions on bone-implantable medical devices, the surface regions having shallow layers including materials that are promoters of bone growth and adhesion. The use of holes in the medical device for containing a therapeutic agent such as for example a BMP is also here disclosed although it is not part of the present invention. The shallow surface layers and the holes are resistant to abrasion and damage during implant into a bone.

Beams of energetic conventional ions, accelerated electrically charged atoms or molecules, are widely utilized to form semiconductor device junctions, to modify surfaces by sputtering, and to modify the properties of thin films. Unlike conventional ions, gas cluster ions are formed from clusters of large numbers (having a typical distribution of several hundreds to several thousands with a mean value of a few thousand) of weakly bound atoms or molecules of materials that are gaseous under conditions of standard temperature and pressure (commonly oxygen, nitrogen, or an inert gas such as argon, for example, but any condensable gas can be used to generate gas cluster ions) with each cluster sharing one or more electrical charges, and which are accelerated together through large electric potential differences (on the order of from about 3 kV to about 70 kV or more) to have high total energies. After gas cluster ions have been formed and accelerated, their charge states may be altered or become altered (even neutralized), and they may fragment or may be induced to fragment into smaller cluster ions or into monomer ions and/or neutralized smaller clusters and neutralized monomers, but they tend to retain the relatively high velocities and energies that result from having been accelerated through large electric potential differences, with the energy being distributed over the fragments. After gas cluster ions have been formed and accelerated, their charge states may be altered or become altered (even neutralized) by collisions with other cluster ions, other neutral clusters, or residual background gas particles, and thus they may fragment or may be induced to fragment into smaller cluster ions or into monomer ions and/or into neutralized smaller clusters and neutralized monomers, but the resulting cluster ions, neutral clusters, and monomer ions and neutral monomers tend to retain the relatively high velocities and energies that result from having been accelerated through large electric potential differences, with the accelerated gas cluster ion energy being distributed over the fragments. As used herein, the terms "GCIB", "gas cluster ion beam" and "gas cluster ion" are intended to encompass not only ionized beams and ions, but also accelerated beams and ions that have had all or a portion of their charge states modified (including neutralized) following their acceleration. The terms "GCIB" and "gas cluster ion beam" are intended to encompass all beams that comprise accelerated gas cluster ions even though they may also comprise non-clustered particles. As used herein, the term "Neutral Beam" is intended to mean a beam of neutral gas clusters and/or neutral monomers derived from an accelerated gas cluster ion beam and wherein the acceleration results from acceleration of a gas cluster ion beam. As used herein, the term "monomer" refers equally to either a single atom or a single molecule. The terms "atom," "molecule," and "monomer" may be used interchangeably and all refer to the appropriate monomer that is characteristic of the gas under discussion (either a component of a cluster, a component of a cluster ion, or an atom or molecule). For example, a monatomic gas like argon may be referred to in terms of atoms, molecules, or monomers and each of those terms means a single atom. Likewise, in the case of a diatomic gas like nitrogen, it may be referred to in terms of atoms, molecules, or monomers, each term meaning a diatomic molecule. Furthermore a molecular gas like CO₂, may be referred to in terms of atoms, molecules, or monomers, each term meaning a three atom molecule, and so forth. These conventions are used to simplify generic discussions of gases and gas clusters or gas cluster ions independent of whether they are monatomic, diatomic, or molecular in their gaseous form.

Because the energies of individual atoms within a gas cluster ion are very small, typically a few eV to some tens of eV, the atoms penetrate through, at most, only a few atomic layers of a target surface during impact. This shallow penetration (typically a few nanometers or less to about ten nanometers, depending on the beam acceleration) of the impacting atoms means all of the energy carried by the entire cluster ion is consequently dissipated in an extremely small volume in a very shallow surface layer during a time period less than a microsecond. This is different from using conventional ion beams where the penetration into the material may be much greater, sometimes several hundred nanometers, producing changes and material modification deep below the surface of the material (depending on ion beam energy). Because of the high total energy of the gas cluster ion and extremely small interaction volume due to shallow penetration, the deposited energy density at the impact site is far greater than in the case of bombardment by conventional ions. Accordingly, at the point of impact of a gas cluster ion on a substrate such as a metal, oxide, or ceramic, there is a momentary (less than a microsecond) high temperature and high pressure transient condition that results in dissociation of the gas cluster and can result in dissociation of molecules, as for example HA or other osteoinductive agent or agents, that may be on the surface of the metal, oxide, or ceramic. The transient extreme conditions can drive the molecular dissociation products and perhaps entire molecules from their positions on the surface into the surface of the metal, oxide, or ceramic substrate in a process referred to as "infusion" or "infusing". The molecules of osteoinductive agent and/or dissociation products of the molecules of osteoinductive agent thereby become embedded in and incorporated into the surface and shallow subsurface of the substrate. The more volatile and less chemically reactive dissociation products and the volatile and unreactive components of the gas cluster ions may tend to escape to a greater degree, while the less volatile and/or more reactive dissociation products tend to become infused (and therefore embedded or partially embedded) into a very shallow surface layer (about 1 to about 10 nanometers thick) of the substrate, with many of the dissociation products exposed at the surface where they are available for chemical reaction with both the substrate surface and with surrounding materials from the surgical site and thus are positioned to be able to promote new bone growth and attachment to the substrate. Such a surface layer is referred to as an "infused surface layer" or a "GCIB infused surface layer". For HA (as an example), dissociation products may include Ca, P, O, and H atoms, as well as larger fragments of the HA molecule. The infused surface may also have its crystallinity modified from that of the original pre-infusion substrate surface by the action of the GCIB cluster impacts, typically resulting in conversion to a more amorphous or less crystalline structure. Accelerated Neutral Beams derived from GCIB share many of the same properties as GCIB in that the formation process endows the monomers and gas clusters in the accelerated Neutral Beam with the same velocity and therefore the same energy per monomer or gas molecule constituent of a neutral gas cluster as is the case per molecule constituent of a gas cluster ion (but without the attendant ionic charge and the disadvantages thereof.) Consequently accelerated Neutral Beams, in many cases have advantages of GCIB without some of the disadvantages.

For this reason, the accelerated Neutral Beam is capable of transforming a titania or zirconia surface which has a thin coating of osteoinductive agent into a surface that is primarily titania or zirconia, but having a very thin infused layer containing for example, Ca, P, O, and H atoms (and/or ions) as well as larger fragments osteoinductive agent molecules, and possibly also embedded and/or partially embedded osteoinductive agent molecules. These infusion products are intimately embedded (wholly and/or partially) in the metal, oxide, or ceramic substrate subsurface to a depth of up to about 10 nanometers and many are exposed at the surface and available for promotion of and attachment to new bone growth. Such a surface is said to be infused with osteoinductive agent. An osteoinductive agent-infused titania or zirconia surface inherits beneficial characteristics of the osteoinductive agents that are infused into the surface, especially so in the case of nutrient materials. A unique characteristic of an osteoinductive agent-infused surface of a surgical implant of for example titania or zirconia is that in addition to the availability of the infusion products at the surface, considerable amounts of the original substrate material (for example titania or zirconia) are also exposed at the surface of the infused region, thus the implant site sees both the availability of the osteoinductive agent and its fragments as well as the biocompatibility features of the titania or zirconia. By controlling the portion of the implant that is coated, more or less of the surface can be processed. In one embodiment, more of the original titania or zirconia is exposed at the surface than is the osteoinductive agent.

The metal, oxide, or ceramic surface can optionally also be provided with small holes that are loaded with a medicine such as BMP or an antibiotic, or other medicine that promotes the effectiveness of a bone implant.

Osteoinductive agent coatings may be applied to a bone-implantable medical device by any of several methods, including for examples, spraying a suspension of ultra-fine particles, spraying a solution, precipitation from solution, dipping, electrostatic deposition, ultrasonic spraying, plasma spraying, and sputter coating. When coating, a conventional masking scheme may be employed to limit deposition to selected locations. A coating thickness of from about 0.01 to about 5 micrometers may be utilized.

In one embodiment, the bone-implantable medical device (or portions of the bone-implantable medical device) may be cleaned by accelerated Neutral Beam or GCIB irradiation prior to applying the osteoinductive agent coating.

After the titania or zirconia (or other material suitable for bone implant - such as, for example polyether ether ketone (PEEK)) has been coated with an osteoinductive agent, it is processed by accelerated Neutral Beam irradiation to form an osteoinductive agent-infused surface.

The titania or zirconia (or other material) surface may have holes, and the holes may additionally be loaded with a therapeutic agent. Holes may be of selected size or sizes and pattern to control the dose of the medicine and the distribution of the medicine on the titania or zirconia surface.

When accelerated gas cluster ions are fully dissociated and neutralized, the resulting neutral monomers will have energies approximately equal to the total energy of the original accelerated gas cluster ion, divided by the number, N_{I}, of monomers that comprised the original gas cluster ion at the time it was accelerated. Such dissociated neutral monomers will have energies on the order of from about 1 eV to tens or even as much as a few thousands of eV, depending on the original accelerated energy of the gas cluster ion and the size of the gas cluster at the time of acceleration.

The present invention may employ a high beam purity method and system for deriving from an accelerated gas cluster ion beam an accelerated neutral gas cluster and/or preferably monomer beam that can be employed for a variety of types of surface and shallow subsurface materials processing and which is capable, for many applications, of superior performance compared to conventional GCIB processing. It can provide well-focused, accelerated, intense neutral monomer beams with particles having energies in the range of from about 1 eV to as much as a few thousand eV. This is an energy range in which it has been heretofore impractical with simple, relatively inexpensive apparatus to form intense neutral beams.

These accelerated Neutral Beams are generated by first forming a conventional accelerated GCIB, then partly or essentially fully dissociating it by methods and operating conditions that do not introduce impurities into the beam, then separating the remaining charged portions of the beam from the neutral portion, and subsequently using the resulting accelerated Neutral Beam for workpiece processing. Depending on the degree of dissociation of the gas cluster ions, the Neutral Beam produced may be a mixture of neutral gas monomers and gas clusters or may essentially consist entirely or almost entirely of neutral gas monomers. It is preferred that the accelerated Neutral Beam is a fully dissociated neutral monomer beam.

An advantage of the Neutral Beams that may be produced by the methods and apparatus of this invention, is that they may be used to process electrically insulating materials without producing damage to the material due to charging of the surfaces of such materials by beam transported charges as commonly occurs for all ionized beams including GCIB. For example, in semiconductor and other electronic applications, ions often contribute to damaging or destructive charging of thin dielectric films such as oxides, nitrides, etc. The use of Neutral Beams can enable successful beam processing of polymer, dielectric, and/or other electrically insulating or high electrical resistivity materials, coatings, and films in other applications where ion beams may produce undesired side effects due to surface or other charging effects. Examples include (without limitation) processing of corrosion inhibiting coatings, and irradiation cross-linking and/or polymerization of organic films. In other examples, Neutral Beam induced modifications of polymer or other dielectric materials (e.g. sterilization, smoothing, improving surface biocompatibility, and improving attachment of and/or control of elution rates of drugs) may enable the use of such materials in medical devices for implant and/or other medical/surgical applications. Further examples include Neutral Beam processing of glass, polymer, and ceramic bio-culture labware and/or environmental sampling surfaces where such beams may be used to improve surface characteristics like, for example, roughness, smoothness, hydrophilicity, and biocompatibility.

Since the parent GCIB, from which accelerated Neutral Beams may be formed by the methods and apparatus of the invention, comprises ions it is readily accelerated to desired energy and is readily focused using conventional ion beam techniques. Upon subsequent dissociation and separation of the charged ions from the neutral particles, the neutral beam particles tend to retain their focused trajectories and may be transported for extensive distances with good effect.

When neutral gas clusters in a jet are ionized by electron bombardment, they become heated and/or excited. This may result in subsequent evaporation of monomers from the ionized gas cluster, after acceleration, as it travels down the beamline. Additionally, collisions of gas cluster ions with background gas molecules in the ionizer, accelerator and beamline regions, also heat and excite the gas cluster ions and may result in additional subsequent evolution of monomers from the gas cluster ions following acceleration. When these mechanisms for evolution of monomers are induced by electron bombardment and/or collision with background gas molecules (and/or other gas clusters) of the same gas from which the GCIB was formed, no contamination is contributed to the beam by the dissociation processes that results in evolving the monomers.

There are other mechanisms that can be employed for dissociating (or inducing evolution of monomers from) gas cluster ions in a GCIB without introducing contamination into the beam. Some of these mechanisms may also be employed to dissociate neutral gas clusters in a neutral gas cluster beam. One mechanism is laser irradiation of the cluster-ion beam using infra-red or other laser energy. Laser-induced heating of the gas cluster ions in the laser irradiated GCIB results in excitement and/or heating of the gas cluster ions and causes subsequent evolution of monomers from the beam. Another mechanism is passing the beam through a thermally heated tube so that radiant thermal energy photons impact the gas cluster ions in the beam. The induced heating of the gas cluster ions by the radiant thermal energy in the tube results in excitement and/or heating of the gas cluster ions and causes subsequent evolution of monomers from the beam. In another mechanism, crossing the gas cluster ion beam by a gas jet of the same gas or mixture as the source gas used in formation of the GCIB (or other non-contaminating gas) results in collisions of monomers of the gas in the gas jet with the gas clusters in the ion beam producing excitement and/or heating of the gas cluster ions in the beam and subsequent evolution of monomers from the excited gas cluster ions. By depending entirely on electron bombardment during initial ionization and/or collisions (with other cluster ions, or with background gas molecules of the same gas(es) as those used to form the GCIB) within the beam and/or laser or thermal radiation and/or crossed jet collisions of non-contaminating gas to produce the GCIB dissociation and/or fragmentation, contamination of the beam by collision with other materials is avoided.

As a neutral gas cluster jet from a nozzle travels through an ionizing region where electrons are directed to ionize the clusters, a cluster may remain un-ionized or may acquire a charge state, q, of one or more charges (by ejection of electrons from the cluster by an incident electron). The ionizer operating conditions influence the likelihood that a gas cluster will take on a particular charge state, with more intense ionizer conditions resulting in greater probability that a higher charge state will be achieved. More intense ionizer conditions resulting in higher ionization efficiency may result from higher electron flux and/or higher (within limits) electron energy. Once the gas cluster has been ionized, it is typically extracted from the ionizer, focused into a beam, and accelerated by falling through an electric field. The amount of acceleration of the gas cluster ion is readily controlled by controlling the magnitude of the accelerating electric field. Typical commercial GCIB processing tools generally provide for the gas cluster ions to be accelerated by an electric field having an adjustable accelerating potential, V_{Acc}, typically of, for example, from about 1kV to 70 kV (but not limited to that range - V_{Acc} up to 200 kV or even more may be feasible). Thus a singly charged gas cluster ion achieves an energy in the range of from 1 to 70 keV (or more if larger V_{Acc} is used) and a multiply charged (for example, without limitation, charge state, q=3 electronic charges) gas cluster ion achieves an energy in the range of from 3 to 210 keV (or more for higher V_{Acc}). For other gas cluster ion charge states and acceleration potentials, the accelerated energy per cluster is qV_{Acc} eV. From a given ionizer with a given ionization efficiency, gas cluster ions will have a distribution of charge states from zero (not ionized) to a higher number such as for example 6 (or with high ionizer efficiency, even more), and the most probable and mean values of the charge state distribution also increase with increased ionizer efficiency (higher electron flux and/or energy). Higher ionizer efficiency also results in increased numbers of gas cluster ions being formed in the ionizer. In many cases, GCIB processing throughput increases when operating the ionizer at high efficiency results in increased GCIB current. A downside of such operation is that multiple charge states that may occur on intermediate size gas cluster ions can increase crater and/or rough interface formation by those ions, and often such effects may operate counterproductively to the intent of the processing. Thus for many GCIB surface processing recipes, selection of the ionizer operating parameters tends to involve more considerations than just maximizing beam current. In some processes, use of a "pressure cell" (see US Pat. 7,060,989, to Swenson et al.) may be employed to permit operating an ionizer at high ionization efficiency while still obtaining acceptable beam processing performance by moderating the beam energy by gas collisions in an elevated pressure "pressure cell."

With the present invention there is no downside to operating the ionizer at high efficiency - in fact such operation is sometimes preferred. When the ionizer is operated at high efficiency, there may be a wide range of charge states in the gas cluster ions produced by the ionizer. This results in a wide range of velocities in the gas cluster ions in the extraction region between the ionizer and the accelerating electrode, and also in the downstream beam. This may result in an enhanced frequency of collisions between and among gas cluster ions in the beam that generally results in a higher degree of fragmentation of the largest gas cluster ions. Such fragmentation may result in a redistribution of the cluster sizes in the beam, skewing it toward the smaller cluster sizes. These cluster fragments retain energy in proportion to their new size (N) and so become less energetic while essentially retaining the accelerated velocity of the initial unfragmented gas cluster ion. The change of energy with retention of velocity following collisions has been experimentally verified (as for example reported in Toyoda, N. et al., "Cluster size dependence on energy and velocity distributions of gas cluster ions after collisions with residual gas," Nucl. Instr. & Meth. in Phys. Research B 257 (2007), pp 662-665). Fragmentation may also result in redistribution of charges in the cluster fragments. Some uncharged fragments likely result and multi-charged gas cluster ions may fragment into several charged gas cluster ions and perhaps some uncharged fragments. It is understood by the inventors that design of the focusing fields in the ionizer and the extraction region may enhance the focusing of the smaller gas cluster ions and monomer ions to increase the likelihood of collision with larger gas cluster ions in the beam extraction region and in the downstream beam, thus contributing to the dissociation and/or fragmenting of the gas cluster ions.

In an embodiment of the present invention, background gas pressure in the ionizer, acceleration region, and beamline may optionally be arranged to have a higher pressure than is normally utilized for good GCIB transmission. This can result in additional evolution of monomers from gas cluster ions (beyond that resulting from the heating and/or excitement resulting from the initial gas cluster ionization event). Pressure may be arranged so that gas cluster ions have a short enough mean-free-path and a long enough flight path between ionizer and workpiece that they must undergo multiple collisions with background gas molecules.

For a homogeneous gas cluster ion containing N monomers and having a charge state of q and which has been accelerated through an electric field potential drop of V_{Acc} volts, the cluster will have an energy of approximately qV_{Acc}/N_{I} eV per monomer, where N_{I} is the number of monomers in the cluster ion at the time of acceleration. Except for the smallest gas cluster ions, a collision of such an ion with a background gas monomer of the same gas as the cluster source gas will result in additional deposition of approximately qV_{Acc}/N_{I} eV into the gas cluster ion. This energy is relatively small compared to the overall gas cluster ion energy (qV_{Acc}) and generally results in excitation or heating of the cluster and in subsequent evolution of monomers from the cluster. It is believed that such collisions of larger clusters with background gas seldom fragment the cluster but rather heats and/or excites it to result in evolution of monomers by evaporation or similar mechanisms. Regardless of the source of the excitation that results in the evolution of a monomer or monomers from a gas cluster ion, the evolved monomer(s) have approximately the same energy per particle, qV_{Acc}/N_{I} eV, and retain approximately the same velocity and trajectory as the gas cluster ion from which they have evolved. When such monomer evolutions occur from a gas cluster ion, whether they result from excitation or heating due to the original ionization event, a collision, or radiant heating, the charge has a high probability of remaining with the larger residual gas cluster ion. Thus after a sequence of monomer evolutions, a large gas cluster ion may be reduced to a cloud of co-traveling monomers with perhaps a smaller residual gas cluster ion (or possibly several if fragmentation has also occurred). The co-traveling monomers following the original beam trajectory all have approximately the same velocity as that of the original gas cluster ion and each has energy of approximately qV_{Acc}/N_{I} eV. For small gas cluster ions, the energy of collision with a background gas monomer is likely to completely and violently dissociate the small gas cluster and it is uncertain whether in such cases the resulting monomers continue to travel with the beam or are ejected from the beam.

Prior to the GCIB reaching the workpiece, the remaining charged particles (gas cluster ions, particularly small and intermediate size gas cluster ions and some charged monomers, but also including any remaining large gas cluster ions) in the beam are separated from the neutral portion of the beam, leaving only a Neutral Beam for processing the workpiece.

In typical operation, the fraction of power in the neutral beam component relative to that in the full (charged plus neutral) beam delivered at the processing target is in the range of from about 5% to 95%, so by the separation methods and apparatus of the present invention it is possible to deliver that portion of the kinetic energy of the full accelerated charged beam to the target as a Neutral Beam.

The dissociation of the gas cluster ions and thus the production of high neutral monomer beam energy is facilitated by 1) Operating at higher acceleration voltages. This increases qV_{Acc}/N for any given cluster size. 2) Operating at high ionizer efficiency. This increases qV_{Acc}/N for any given cluster size by increasing q and increases cluster-ion on cluster-ion collisions in the extraction region due to the differences in charge states between clusters; 3) Operating at a high ionizer, acceleration region, or beamline pressure or operating with a gas jet crossing the beam, or with a longer beam path, all of which increase the probability of background gas collisions for a gas cluster ion of any given size; 4) Operating with laser irradiation or thermal radiant heating of the beam, which directly promote evolution of monomers from the gas cluster ions; and 5) Operating at higher nozzle gas flow, which increases transport of gas, clustered and perhaps unclustered into the GCIB trajectory, which increases collisions resulting in greater evolution of monomers.

Measurement of the Neutral Beam cannot be made by current measurement as is convenient for gas cluster ion beams. A Neutral Beam power sensor is used to facilitate dosimetry when irradiating a workpiece with a Neutral Beam. The Neutral Beam sensor is a thermal sensor that intercepts the beam (or optionally a known sample of the beam). The rate of rise of temperature of the sensor is related to the energy flux resulting from energetic beam irradiation of the sensor. The thermal measurements must be made over a limited range of temperatures of the sensor to avoid errors due to thermal re-radiation of the energy incident on the sensor. For a GCIB process, the beam power (watts) is equal to the beam current (amps) times V_{Acc}, the beam acceleration voltage. When a GCIB irradiates a workpiece for a period of time (seconds), the energy (joules) received by the workpiece is the product of the beam power and the irradiation time. The processing effect of such a beam when it processes an extended area is distributed over the area (for example, cm²). For ion beams, it has been conveniently conventional to specify a processing dose in terms of irradiated ions/cm², where the ions are either known or assumed to have at the time of acceleration an average charge state, q, and to have been accelerated through a potential difference of, V_{Acc} volts, so that each ion carries an energy of q V_{Acc} eV (an eV is approximately 1.6 x 10⁻¹⁹ joule). Thus an ion beam dose for an average charge state, q, accelerated by V_{Acc} and specified in ions/cm² corresponds to a readily calculated energy dose expressible in joules/cm². For an accelerated Neutral Beam derived from an accelerated GCIB as utilized in the present invention, the value of q at the time of acceleration and the value of V_{Acc} is the same for both of the (later- formed and separated) charged and uncharged fractions of the beam. The power in the two (neutral and charged) fractions of the GCIB divides proportional to the mass in each beam fraction. Thus for the accelerated Neutral Beam as employed in the invention, when equal areas are irradiated for equal times, the energy dose (joules/cm²) deposited by the Neutral Beam is necessarily less than the energy dose deposited by the full GCIB. By using a thermal sensor to measure the power in the full GCIB P_{G} and that in the Neutral Beam P_{N} (which is commonly found to be about 5% to 95% that of the full GCIB) it is possible to calculate a compensation factor for use in the Neutral Beam processing dosimetry. When P_{N} is aP_{G}, then the compensation factor is, k = 1/a. Thus if a workpiece is processed using a Neutral Beam derived from a GCIB, for a time duration is made to be k times greater than the processing duration for the full GCIB (including charged and neutral beam portions) required to achieve a dose of D ions/cm², then the energy doses deposited in the workpiece by both the Neutral Beam and the full GCIB are the same (though the results may be different due to qualitative differences in the processing effects due to differences of particle sizes in the two beams.) As used herein, a Neutral Beam process dose compensated in this way is sometimes described as having an energy/cm² equivalence of a dose of D ions/cm².

Use of a Neutral Beam derived from a gas cluster ion beam in combination with a thermal power sensor for dosimetry in many cases has advantages compared with the use of the full gas cluster ion beam or an intercepted or diverted portion, which inevitably comprises a mixture of gas cluster ions and neutral gas clusters and/or neutral monomers, and which is conventionally measured for dosimetry purposes by using a beam current measurement. Some advantages are as follows:
1) The dosimetry can be more precise with the Neutral Beam using a thermal sensor for dosimetry because the total power of the beam is measured. With a GCIB employing the traditional beam current measurement for dosimetry, only the contribution of the ionized portion of the beam is measured and employed for dosimetry. Minute-to-minute and setup-to-setup changes to operating conditions of the GCIB apparatus may result in variations in the fraction of neutral monomers and neutral clusters in the GCIB. These variations can result in process variations that may be less controlled when the dosimetry is done by beam current measurement.
2) With a Neutral Beam, any material may be processed, including highly insulating materials and other materials that may be damaged by electrical charging effects, without the necessity of providing a source of target neutralizing electrons to prevent workpiece charging due to charge transported to the workpiece by an ionized beam. When employed with conventional GCIB, target neutralization to reduce charging is seldom perfect, and the neutralizing electron source itself often introduces problems such as workpiece heating, contamination from evaporation or sputtering in the electron source, etc. Since a Neutral Beam does not transport charge to the workpiece, such problems are reduced.
3) There is no necessity for an additional device such as a large aperture high strength magnet to separate energetic monomer ions from the Neutral Beam. In the case of conventional GCIB the risk of energetic monomer ions (and other small cluster ions) being transported to the workpiece, where they penetrate producing deep damage, is significant and an expensive magnetic filter is routinely required to separate such particles from the beam. In the case of the Neutral Beam apparatus of the invention, the separation of all ions from the beam to produce the Neutral Beam inherently removes all monomer ions.

One embodiment of the present invention provides a method of modifying a surface of a bone-implantable medical device comprising the steps of: coating at least a first portion of the surface of the medical device with an osteoinductive agent to form a coated surface region; and first irradiating at least a portion of the coated surface region with a first accelerated Neutral Beam. The first irradiating step may form a shallow surface and subsurface layer comprising embedded molecules and/or dissociation products of the osteoinductive agent. The first accelerated Neutral Beam may be derived from a first gas cluster ion beam and the shallow surface and subsurface layer may be an infused surface layer. The osteoinductive agent may comprise, separately or in combination, any of the materials from the group consisting of: a nutrient material, tricalcium phosphate, hydroxyapatite, Bioglass 45S5, Bioglass 58S, a bone growth-stimulating agent, a growth factor, a cytokine, a TGF-β protein, a BMP, a GPI-anchored signaling protein, an RGM, and a growth regulatory protein. The surface of the bone-implantable medical device may comprise, separately or in combination, any of a metal, an oxide, or a ceramic. The surface may comprise titanium, titania, or zirconia.

The method may further comprise the steps, prior to the coating step: forming a second ion beam that is a gas cluster ion beam; and second irradiating at least a second portion of the surface of the medical device to clean the at least a second portion of the surface. The method may further comprise the steps, prior to the coating step: forming a second accelerated Neutral Beam; and second irradiating at least a second portion of the surface of the medical device to clean the at least a second portion of the surface. The first irradiating step may comprise employing a mask to control the at least a portion of the coated surface region that is irradiated. The first irradiating step may comprise positioning the medical device with respect to the first accelerated Neutral Beam to control the at least a portion of the coated surface region that is irradiated.

The method may further comprise the steps of: forming one or more holes in the surface of the medical device; loading at least one of the one or more holes with a therapeutic agent; and third irradiating an exposed surface of the therapeutic agent in at least one loaded hole with a third accelerated Neutral Beam to form a barrier layer at the exposed surface. The third accelerated Neutral Beam may be derived from an accelerated gas cluster ion beam. The barrier layer may control an elution rate of therapeutic agent. The barrier layer may control a rate of inward diffusion of a fluid into the hole.

Another embodiment (not part of the present invention) provides a method of modifying a surface of a bone-implantable medical device comprising the steps of: forming one or more holes in the surface of the medical device; first loading at least one of the one or more holes with a first therapeutic agent; and first irradiating an exposed surface of the first therapeutic agent in at least one loaded hole with a first accelerated Neutral Beam to form a first barrier layer at the exposed surface in the at least one loaded hole. The one or more holes may be disposed on the surface in a predetermined pattern to distribute the first therapeutic agent on the surface according to a predetermined distribution plan. At least one of the one or more holes may be loaded with a second therapeutic agent different from the first therapeutic agent. At least one of the one or more holes may be loaded with a first quantity of the first therapeutic agent that differs from a second quantity of the first therapeutic agent loaded in at least another of the one or more holes.

The first loading step may not completely fill the at least one hole, and following the first irradiating step further comprising the steps of: second loading the at least one incompletely filled hole with a second therapeutic agent overlying the first barrier layer; and second irradiating an exposed surface of the second therapeutic agent in at least one second loaded hole with a second accelerated Neutral Beam to form a second barrier layer at the exposed surface in the at least one loaded hole. The first barrier layer and the second barrier layer may have different properties for controlling elution rate of the first and second therapeutic agents. The first accelerated Neutral Beam may be derived from a first gas cluster ion beam and the second accelerated Neutral Beam may be derived from a second gas cluster ion beam. The first accelerated Neutral Beam may be derived from a first gas cluster ion beam.

Yet another embodiment of the present invention provides a bone-implantable medical device having a surface, wherein at least a portion of the surface comprises a shallow layer comprising molecules and/or dissociation products of an osteoinductive agent embedded into the at least a portion of the surface. The shallow layer may be an accelerated Neutral Beam infused surface layer. The surface of the medical device may comprise titanium, titania, or zirconia. The medical device may further comprise: one or more holes containing a therapeutic agent in the surface of the medical device; and at least one or more barrier layers at one or more surfaces of the therapeutic agent contained in the one or more holes; wherein the at least one or more barrier layers control at least one elution rate of therapeutic agent.

Still another embodiment (which is not part of the present invention) provides a bone-implantable medical device having a surface, wherein at least a portion of the surface comprises: one or more holes containing a therapeutic agent in the surface of the medical device; and at least one or more barrier layers at one or more surfaces of the therapeutic agent contained in the one or more holes; wherein the at least one or more barrier layers control at least one elution rate of therapeutic agent. The surface of the medical device may comprise titanium, titania, or zirconia. The one or more holes one may be disposed on the surface in a predetermined pattern to distribute the therapeutic agent on the surface according to a predetermined distribution plan.

### BRIEF DESCRIPTION OF THE DRAWINGS

For a better understanding of the present invention, together with other and further objects thereof, reference is made to the accompanying drawings, wherein:
Figure 1 is a schematic illustrating elements of a GCIB processing apparatus 1100 for processing a workpiece using a GCIB;
Figure 2 is a schematic illustrating elements of another GCIB processing apparatus 1200 for workpiece processing using a GCIB, wherein scanning of the ion beam and manipulation of the workpiece is employed;
Figure 3 is a schematic of a Neutral Beam processing apparatus 1300 according to an embodiment of the invention, which uses electrostatic deflection plates to separate the charged and uncharged beams;
Figure 4 is a schematic of a Neutral Beam processing apparatus 1400 according to an embodiment of the invention, using a thermal sensor for Neutral Beam measurement;
Figures 5A and 5B show comparison of a drug coating on a cobalt-chrome coupon representing a drug eluting medical device, wherein processing with a Neutral Beam produces a superior result to processing with a full GCIB;
Figure 6 is a view of a prior art bone-implantable medical device, a dental implant device;
Figure 7A is a view of a dental implant device with holes for loading an osteoinductive agent or other medicine as may be employed in embodiments of the invention;
Figure 7B is a view of a dental implant device with selected portions of its surface coated with an osteoinductive agent according to an embodiment of the invention;
Figure 7C shows an ion beam irradiation step in the formation of a GCIB infused layer in portions of the surface of a dental implant device according to an embodiment of the invention;
Figure 7D shows a view of a dental implant device having a surface with an infused osteoinductive agent according to an embodiment of the invention;
Figure 7E shows a view of a dental implant device with an infused osteoinductive agent on a portion thereof, further having holes that are loaded with a medicine and/or an osteoinductive agent according to an embodiment of the invention;
Figure 7F shows a GCIB irradiation step in the formation of a thin barrier layer in the surface of therapeutic agent loaded in holes in a dental implant device according to an embodiment of the invention;
Figures 8A, 8B, 8C, and 8D show detail of the steps for loading a hole in a bone-implantable medical device with a therapeutic agent, and forming a thin barrier layer thereon using ion beam irradiation according to embodiments of the invention;
Figure 9 shows a bone-implantable hip joint prosthesis employing embodiments of the invention; and
Figure 10 shows a cross sectional view of the surface of a bone-implantable medical device having a variety of therapeutic agent-loaded holes which is not part of the invention.

### DETAILED DESCRIPTION OF THE PREFERRED EMBODIMENTS

In the following description, for simplification, item numbers from earlier-described figures may appear in subsequently-described figures without discussion. Likewise, items discussed in relation to earlier figures may appear in subsequent figures without item numbers or additional description. In such cases items with like numbers are like items and have the previously-described features and functions, and illustration of items without item numbers shown in the present figure refer to like items having the same functions as the like items illustrated in earlier-discussed numbered figures.

In an embodiment of the invention, a Neutral Beam derived from an accelerated gas cluster ion beam is employed to process insulating (and other sensitive) surfaces.

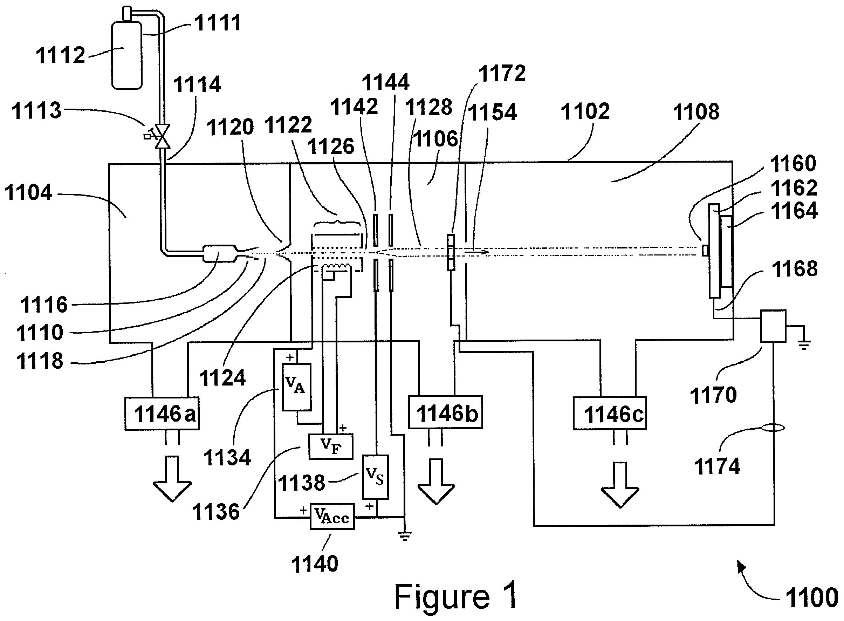

Reference is now made to Figure 1, which shows a schematic configuration for a GCIB processing apparatus 1100. A low-pressure vessel 1102 has three fluidly connected chambers: a nozzle chamber 1104, an ionization/acceleration chamber 1106, and a processing chamber 1108. The three chambers are evacuated by vacuum pumps 1146a, 1146b, and 1146c, respectively. A pressurized condensable source gas 1112 (for example argon) stored in a gas storage cylinder 1111 flows through a gas metering valve 1113 and a feed tube 1114 into a stagnation chamber 1116. Pressure (typically a few atmospheres) in the stagnation chamber 1116 results in ejection of gas into the substantially lower pressure vacuum through a nozzle 1110, resulting in formation of a supersonic gas jet 1118. Cooling, resulting from the expansion in the jet, causes a portion of the gas jet 1118 to condense into clusters, each consisting of from several to several thousand weakly bound atoms or molecules. A gas skimmer aperture 1120 is employed to control flow of gas into the downstream chambers by partially separating gas molecules that have not condensed into a cluster jet from the cluster jet. Excessive pressure in the downstream chambers can be detrimental by interfering with the transport of gas cluster ions and by interfering with management of the high voltages that may be employed for beam formation and transport. Suitable condensable source gases 1112 include, but are not limited to argon and other condensable noble gases, nitrogen, carbon dioxide, oxygen, and many other gases and/or gas mixtures. After formation of the gas clusters in the supersonic gas jet 1118, at least a portion of the gas clusters are ionized in an ionizer 1122 that is typically an electron impact ionizer that produces electrons by thermal emission from one or more incandescent filaments 1124 (or from other suitable electron sources) and accelerates and directs the electrons, enabling them to collide with gas clusters in the gas jet 1118. Electron impacts with gas clusters eject electrons from some portion of the gas clusters, causing those clusters to become positively ionized. Some clusters may have more than one electron ejected and may become multiply ionized. Control of the number of electrons and their energies after acceleration typically influences the number of ionizations that may occur and the ratio between multiple and single ionizations of the gas clusters. A suppressor electrode 1142, and grounded electrode 1144 extract the cluster ions from the ionizer exit aperture 1126, accelerate them to a desired energy (typically with acceleration potentials of from several hundred V to several tens of kV), and focuses them to form a GCIB 1128. The region that the GCIB 1128 traverses between the ionizer exit aperture 126 and the suppressor electrode 1142 is referred to as the extraction region. The axis (determined at the nozzle 1110), of the supersonic gas jet 1118 containing gas clusters is substantially the same as the axis 1154 of the GCIB 1128. Filament power supply 1136 provides filament voltage V_{f} to heat the ionizer filament 1124. Anode power supply 1134 provides anode voltage V_{A} to accelerate thermoelectrons emitted from filament 1124 to cause the thermoelectrons to irradiate the cluster-containing gas jet 1118 to produce cluster ions. A suppression power supply 1138 supplies suppression voltage V_{S} (on the order of several hundred to a few thousand volts) to bias suppressor electrode 1142. Accelerator power supply 1140 supplies acceleration voltage V_{Acc} to bias the ionizer 1122 with respect to suppressor electrode 1142 and grounded electrode 1144 so as to result in a total GCIB acceleration potential equal to V_{Acc}. Suppressor electrode 1142 serves to extract ions from the ionizer exit aperture 1126 of ionizer 1122 and to prevent undesired electrons from entering the ionizer 1122 from downstream, and to form a focused GCIB 1128.

A workpiece 1160, which may (for example) be a medical device, a semiconductor material, an optical element, or other workpiece to be processed by GCIB processing, is held on a workpiece holder 1162, which disposes the workpiece in the path of the GCIB 1128. The workpiece holder is attached to but electrically insulated from the processing chamber 1108 by an electrical insulator 1164. Thus, GCIB 1128 striking the workpiece 1160 and the workpiece holder 1162 flows through an electrical lead 1168 to a dose processor 1170. A beam gate 1172 controls transmission of the GCIB 1128 along axis 1154 to the workpiece 1160. The beam gate 1172 typically has an open state and a closed state that is controlled by a linkage 1174 that may be (for example) electrical, mechanical, or electromechanical. Dose processor 1170 controls the open/closed state of the beam gate 1172 to manage the GCIB dose received by the workpiece 1160 and the workpiece holder 1162. In operation, the dose processor 1170 opens the beam gate 1172 to initiate GCIB irradiation of the workpiece 1160. Dose processor 1170 typically integrates GCIB electrical current arriving at the workpiece 1160 and workpiece holder 1162 to calculate an accumulated GCIB irradiation dose. At a predetermined dose, the dose processor 1170 closes the beam gate 1172, terminating processing when the predetermined dose has been achieved.

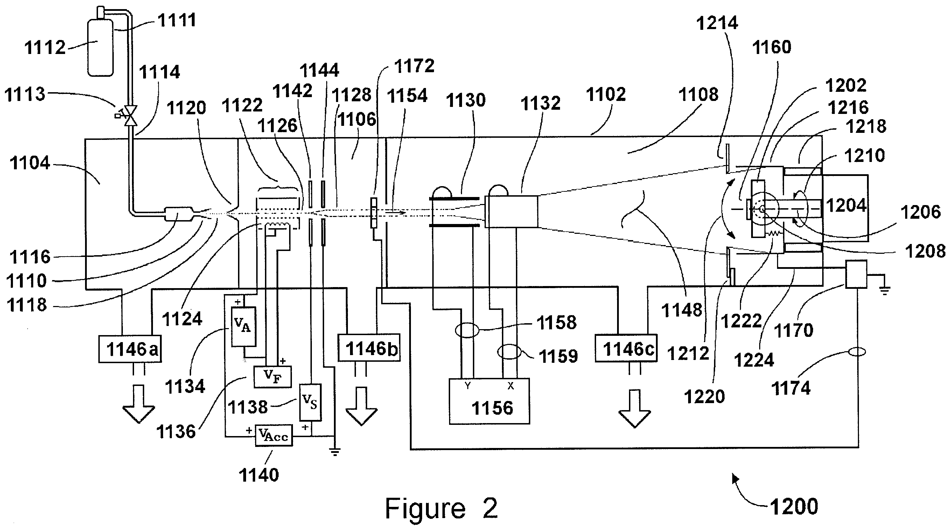

Figure 2 shows a schematic illustrating elements of another GCIB processing apparatus 1200 for workpiece processing using a GCIB, wherein scanning of the ion beam and manipulation of the workpiece is employed. A workpiece 1160 to be processed by the GCIB processing apparatus 1200 is held on a workpiece holder 1202, disposed in the path of the GCIB 1128. In order to accomplish uniform processing of the workpiece 1160, the workpiece holder 1202 is designed to manipulate workpiece 1160, as may be required for uniform processing.

Any workpiece surfaces that are non-planar, for example, spherical or cup-like, rounded, irregular, or other un-flat configuration, may be oriented within a range of angles with respect to the beam incidence to obtain optimal GCIB processing of the workpiece surfaces. The workpiece holder 1202 can be fully articulated for orienting all non-planar surfaces to be processed in suitable alignment with the GCIB 1128 to provide processing optimization and uniformity. More specifically, when the workpiece 1160 being processed is non-planar, the workpiece holder 1202 may be rotated in a rotary motion 1210 and articulated in articulation motion 1212 by an articulation/rotation mechanism 1204. The articulation/rotation mechanism 1204 may permit 360 degrees of device rotation about longitudinal axis 1206 (which is coaxial with the axis 1154 of the GCIB 1128) and sufficient articulation about an axis 1208 perpendicular to axis 1206 to maintain the workpiece surface to within a desired range of beam incidence.

Under certain conditions, depending upon the size of the workpiece 1160, a scanning system may be desirable to produce uniform irradiation of a large workpiece. Although often not necessary for GCIB processing, two pairs of orthogonally oriented electrostatic scan plates 1130 and 1132 may be utilized to produce a raster or other scanning pattern over an extended processing area. When such beam scanning is performed, a scan generator 1156 provides X-axis scanning signal voltages to the pair of scan plates 1132 through lead pair 1159 and Y-axis scanning signal voltages to the pair of scan plates 1130 through lead pair 1158. The scanning signal voltages are commonly triangular waves of different frequencies that cause the GCIB 1128 to be converted into a scanned GCIB 1148, which scans the entire surface of the workpiece 1160. A scanned beam-defining aperture 1214 defines a scanned area. The scanned beam-defining aperture 1214 is electrically conductive and is electrically connected to the low-pressure vessel 1102 wall and supported by support member 1220. The workpiece holder 1202 is electrically connected via a flexible electrical lead 1222 to a faraday cup 1216 that surrounds the workpiece 1160 and the workpiece holder 1202 and collects all the current passing through the defining aperture 1214. The workpiece holder 1202 is electrically isolated from the articulation/rotation mechanism 1204 and the faraday cup 1216 is electrically isolated from and mounted to the low-pressure vessel 1102 by insulators 1218. Accordingly, all current from the scanned GCIB 1148, which passes through the scanned beam-defining aperture 1214 is collected in the faraday cup 1216 and flows through electrical lead 1224 to the dose processor 1170. In operation, the dose processor 1170 opens the beam gate 1172 to initiate GCIB irradiation of the workpiece 1160. The dose processor 1170 typically integrates GCIB electrical current arriving at the workpiece 1160 and workpiece holder 1202 and faraday cup 1216 to calculate an accumulated GCIB irradiation dose per unit area. At a predetermined dose, the dose processor 1170 closes the beam gate 1172, terminating processing when the predetermined dose has been achieved. During the accumulation of the predetermined dose, the workpiece 1160 may be manipulated by the articulation/rotation mechanism 1204 to ensure processing of all desired surfaces.

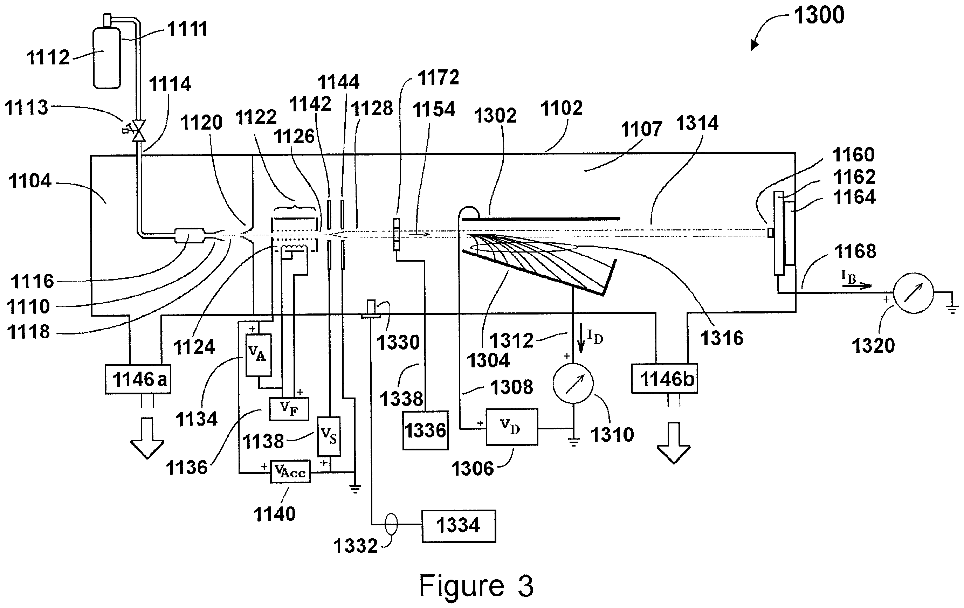

Figure 3 is a schematic of a Neutral Beam processing apparatus 1300 of an exemplary type that may be employed for Neutral Beam processing according to embodiments of the invention. It uses electrostatic deflection plates to separate the charged and uncharged portions of a GCIB. A beamline chamber 1107 encloses the ionizer and accelerator regions and the workpiece processing regions. The beamline chamber 1107 has high conductance and so the pressure is substantially uniform throughout. A vacuum pump 1146b evacuates the beamline chamber 1107. Gas flows into the beamline chamber 1107 in the form of clustered and unclustered gas transported by the gas jet 1118 and in the form of additional unclustered gas that leaks through the gas skimmer aperture 1120. A pressure sensor 1330 transmits pressure data from the beamline chamber 1107 through an electrical cable 1332 to a pressure sensor controller 1334, which measures and displays pressure in the beamline chamber 1107. The pressure in the beamline chamber 1107 depends on the balance of gas flow into the beamline chamber 1107 and the pumping speed of the vacuum pump 1146b. By selection of the diameter of the gas skimmer aperture 1120, the flow of source gas 1112 through the nozzle 1110, and the pumping speed of the vacuum pump 1146b, the pressure in the beamline chamber 1107 equilibrates at a pressure, P_{B}, determined by design and by nozzle flow. The beam flight path from grounded electrode 1144 to workpiece holder 162, is for example, 100 cm. By design and adjustment P_{B} may be approximately 6 x 10⁻⁵ torr (8 x 10⁻³ pascal). Thus the product of pressure and beam path length is approximately 6 x 10⁻³ torr-cm (0.8 pascal-cm) and the gas target thickness for the beam is approximately 1.94 x 10¹⁴ gas molecules per cm², which is observed to be effective for dissociating the gas cluster ions in the GCIB 1128. V_{Acc} may be for example 30kV and the GCIB 1128 is accelerated by that potential. A pair of deflection plates (1302 and 1304) is disposed about the axis 1154 of the GCIB 1128. A deflector power supply 1306 provides a positive deflection voltage V_{D} to deflection plate 1302 via electrical lead 1308. Deflection plate 1304 is connected to electrical ground by electrical lead 1312 and through current sensor/display 1310. Deflector power supply 1306 is manually controllable. V_{D} may be adjusted from zero to a voltage sufficient to completely deflect the ionized portion 1316 of the GCIB 1128 onto the deflection plate 1304 (for example a few thousand volts). When the ionized portion 1316 of the GCIB 1128 is deflected onto the deflection plate 1304, the resulting current, I_{D} flows through electrical lead 1312 and current sensor/display 1310 for indication. When V_{D} is zero, the GCIB 1128 is undeflected and travels to the workpiece 1160 and the workpiece holder 1162. The GCIB beam current I_{B} is collected on the workpiece 1160 and the workpiece holder 1162 and flows through electrical lead 1168 and current sensor/display 1320 to electrical ground. I_{B} is indicated on the current sensor/display 1320. A beam gate 1172 is controlled through a linkage 1338 by beam gate controller 1336. Beam gate controller 1336 may be manual or may be electrically or mechanically timed by a preset value to open the beam gate 1172 for a predetermined interval. In use, V_{D} is set to zero, the beam current, I_{B}, striking the workpiece holder is measured. Based on previous experience for a given GCIB process recipe, an initial irradiation time for a given process is determined based on the measured current, I_{B}. V_{D} is increased until all measured beam current is transferred from I_{B} to I_{D} and I_{D} no longer increases with increasing V_{D}. At this point a Neutral Beam 1314 comprising energetic dissociated components of the initial GCIB 1128 irradiates the workpiece holder 1162. The beam gate 1172 is then closed and the workpiece 1160 placed onto the workpiece holder 1162 by conventional workpiece loading means (not shown). The beam gate 1172 is opened for the predetermined initial radiation time. After the irradiation interval, the workpiece may be examined and the processing time adjusted as necessary to calibrate the duration of Neutral Beam processing based on the measured GCIB beam current I_{B}. Following such a calibration process, additional workpieces may be processed using the calibrated exposure duration.

The Neutral Beam 1314 contains a repeatable fraction of the initial energy of the accelerated GCIB 1128. The remaining ionized portion 1316 of the original GCIB 1128 has been removed from the Neutral Beam 1314 and is collected by the grounded deflection plate 1304. The ionized portion 1316 that is removed from the Neutral Beam 1314 may include monomer ions and gas cluster ions including intermediate size gas cluster ions. Because of the monomer evaporation mechanisms due to cluster heating during the ionization process, intra-beam collisions, background gas collisions, and other causes (all of which result in erosion of clusters) the Neutral Beam substantially consists of neutral monomers, while the separated charged particles are predominately cluster ions. The inventors have confirmed this by suitable measurements that include re-ionizing the Neutral Beam and measuring the charge to mass ratio of the resulting ions. As will be shown below, certain superior process results are obtained by processing workpieces using this Neutral Beam.

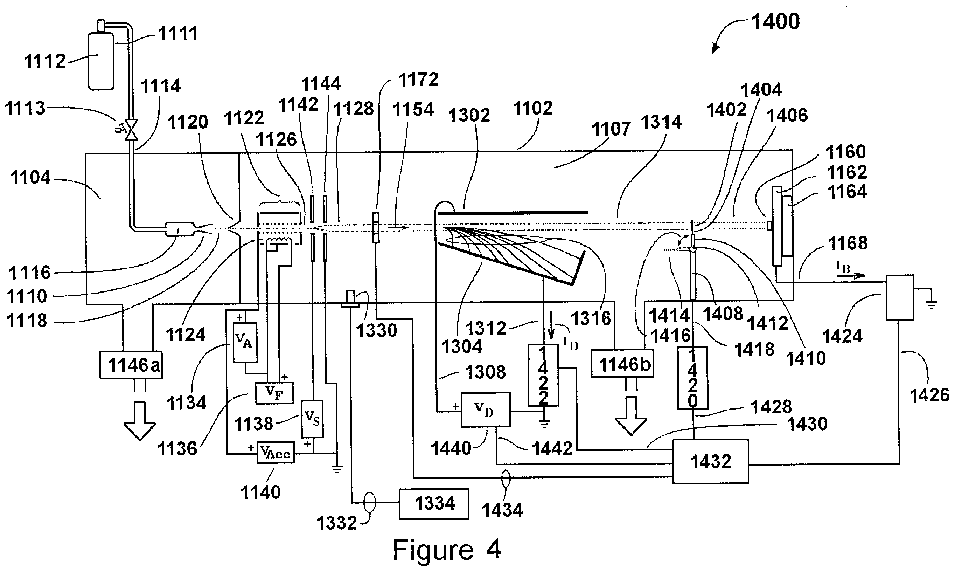

Figure 4 is a schematic of a Neutral Beam processing apparatus 1400 as may, for example, be used in generating Neutral Beams as may be employed in embodiments of the invention. It uses a thermal sensor for Neutral Beam measurement. A thermal sensor 1402 attaches via low thermal conductivity attachment 1404 to a rotating support arm 1410 attached to a pivot 1412. Actuator 1408 moves thermal sensor 1402 via a reversible rotary motion 1416 between positions that intercept the Neutral Beam 1314 or GCIB 1128 and a parked position indicated by 1414 where the thermal sensor 1402 does not intercept any beam. When thermal sensor 1402 is in the parked position (indicated by 1414) the GCIB 1128 or Neutral Beam 1314 continues along path 1406 for irradiation of the workpiece 1160 and/or workpiece holder 1162. A thermal sensor controller 1420 controls positioning of the thermal sensor 1402 and performs processing of the signal generated by thermal sensor 1402. Thermal sensor 1402 communicates with the thermal sensor controller 1420 through an electrical cable 1418. Thermal sensor controller 1420 communicates with a dosimetry controller 1432 through an electrical cable 1428. A beam current measurement device 1424 measures beam current I_{B} flowing in electrical lead 1168 when the GCIB 1128 strikes the workpiece 1160 and/or the workpiece holder 1162. Beam current measurement device 1424 communicates a beam current measurement signal to dosimetry controller 1432 via electrical cable 1426. Dosimetry controller 1432 controls setting of open and closed states for beam gate 1172 by control signals transmitted via linkage 1434. Dosimetry controller 1432 controls deflector power supply 1440 via electrical cable 1442 and can control the deflection voltage V_{D} between voltages of zero and a positive voltage adequate to completely deflect the ionized portion 1316 of the GCIB 1128 to the deflection plate 1304. When the ionized portion 1316 of the GCIB 1128 strikes deflection plate 1304, the resulting current I_{D} is measured by current sensor 1422 and communicated to the dosimetry controller 1432 via electrical cable 1430. In operation dosimetry controller 1432 sets the thermal sensor 1402 to the parked position 1414, opens beam gate 1172, sets V_{D} to zero so that the full GCIB 1128 strikes the workpiece holder 1162 and/or workpiece 1160. The dosimetry controller 1432 records the beam current I_{B} transmitted from beam current measurement device 1424. The dosimetry controller 1432 then moves the thermal sensor 1402 from the parked position 1414 to intercept the GCIB 1128 by commands relayed through thermal sensor controller 1420. Thermal sensor controller 1420 measures the beam energy flux of GCIB 1128 by calculation based on the heat capacity of the sensor and measured rate of temperature rise of the thermal sensor 1402 as its temperature rises through a predetermined measurement temperature (for example 70 degrees C) and communicates the calculated beam energy flux to the dosimetry controller 1432 which then calculates a calibration of the beam energy flux as measured by the thermal sensor 1402 and the corresponding beam current measured by the beam current measurement device 1424. The dosimetry controller 1432 then parks the thermal sensor 1402 at parked position 1414, allowing it to cool and commands application of positive V_{D} to deflection plate 1302 until all of the current I_{D} due to the ionized portion of the GCIB 1128 is transferred to the deflection plate 1304. The current sensor 1422 measures the corresponding I_{D} and communicates it to the dosimetry controller 1432. The dosimetry controller also moves the thermal sensor 1402 from parked position 1414 to intercept the Neutral Beam 1314 by commands relayed through thermal sensor controller 420. Thermal sensor controller 420 measures the beam energy flux of the Neutral Beam 1314 using the previously determined calibration factor and the rate of temperature rise of the thermal sensor 1402 as its temperature rises through the predetermined measurement temperature and communicates the Neutral Beam energy flux to the dosimetry controller 1432. The dosimetry controller 1432 calculates a neutral beam fraction, which is the ratio of the thermal measurement of the Neutral Beam 1314 energy flux to the thermal measurement of the full GCIB 1128 energy flux at sensor 1402. Under typical operation, a neutral beam fraction of from about 5% to about 95% is achieved. Before beginning processing, the dosimetry controller 1432 also measures the current, I_{D}, and determines a current ratio between the initial values of I_{B} and I_{D}. During processing, the instantaneous I_{D} measurement multiplied by the initial I_{B}/I_{D} ratio may be used as a proxy for continuous measurement of the I_{B} and employed for dosimetry during control of processing by the dosimetry controller 1432. Thus the dosimetry controller 1432 can compensate any beam fluctuation during workpiece processing, just as if an actual beam current measurement for the full GCIB 1128 were available. The dosimetry controller uses the neutral beam fraction to compute a desired processing time for a particular beam process. During the process, the processing time can be adjusted based on the calibrated measurement of I_{D} for correction of any beam fluctuation during the process.

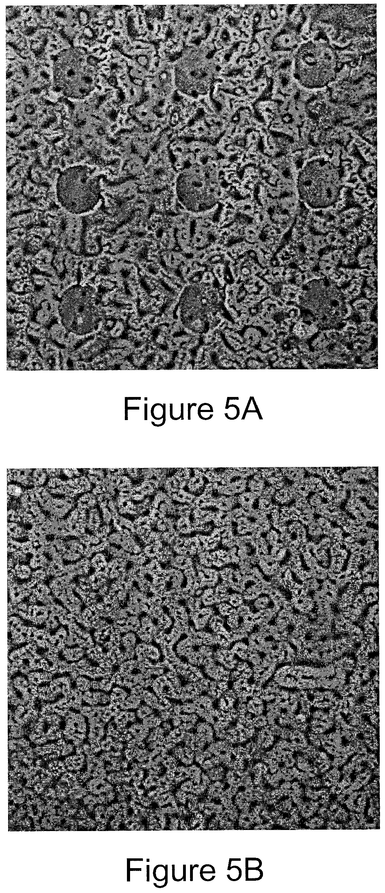

Figures 5A and 5B show comparative results of full GCIB and Neutral Beam processing of a drug film deposited on a cobalt-chrome coupon used to evaluate drug elution rate for a drug eluting coronary stent. Figure 5A represents a sample irradiated using an argon GCIB (including the charged and neutral components) accelerated using V_{Acc} of 30kV with an irradiated dose of 2 x 10¹⁵ gas cluster ions per cm². Figure 5B represents a sample irradiated using a Neutral Beam derived from an argon GCIB accelerated using V_{Acc} of 30kV. The Neutral Beam was irradiated with a thermal energy dose equivalent to that of a 30kV accelerated , 2 x 10¹⁵ gas cluster ion per cm² dose (equivalent determined by beam thermal energy flux sensor). The irradiation for both samples was performed through a cobalt chrome proximity mask having an array of circular apertures of approximately 50 microns diameter for allowing beam transmission. Figure 5A is a scanning electron micrograph of a 300 micron by 300 micron region of the sample that was irradiated through the mask with full beam. Figure 5B is a scanning electron micrograph of a 300 micron by 300 micron region of the sample that was irradiated through the mask with a Neutral Beam. The sample shown in Figure 5A exhibits damage and etching caused by the full beam where it passed through the mask. The sample shown in Figure 5B exhibits no visible effect. In elution rate tests in physiological saline solution, the samples processed like the Figure B sample (but without mask) exhibited superior (delayed) elution rate compared to the samples processed like the Figure 5A sample (but without mask). The results support the conclusion that processing with the Neutral Beam contributes to the desired delayed elution effect, while processing with the full GCIB (charged plus neutral components) contributes to weight loss of the drug by etching, with inferior (less delayed) elution rate effect.

To further illustrate the ability of an accelerated Neutral Beam derived from an accelerated GCIB to aid in attachment of a drug to a surface and to provide drug modification in such a way that it produces a barrier layer resulting in delayed drug elution, an additional test was performed. Silicon coupons approximately 1cm by 1cm (1 cm2) were prepared from highly polished clean semiconductor-quality silicon wafers for use as drug deposition substrates. A solution of the drug Rapamycin (Catalog number R-5000, LC Laboratories, Woburn, MA 01801, USA) was formed by dissolving 500mg of Rapamycin in 20ml of acetone. A pipette was then used to dispense approximately 5 micro-liter droplets of the drug solution onto each coupon. Following atmospheric evaporation and vacuum drying of the solution, this left approximately 5mm diameter circular Rapamycin deposits on each of the silicon coupons. Coupons were divided into groups and either left un-irradiated (controls) or irradiated with various conditions of Neutral Beam irradiation. The groups were then placed in individual baths (bath per coupon) of human plasma for 4.5 hours to allow elution of the drug into the plasma. After 4.5 hours, the coupons were removed from the plasma baths, rinsed in deionized water and vacuum dried. Weight measurements were made at the following stages in the process: 1) pre-deposition clean silicon coupon weight; 2) following deposition and drying, weight of coupon plus deposited drug; 3) post-irradiation weight; and 4) post plasma-elution and vacuum drying weight. Thus for each coupon the following information is available: 1) initial weight of the deposited drug load on each coupon; 2) the weight of drug lost during irradiation of each coupon; and 3) the weight of drug lost during plasma elution for each coupon. For each irradiated coupon it was confirmed that drug loss during irradiation was negligible. Drug loss during elution in human plasma is shown in Table 1. The groups were as follows: Control Group - no irradiation was performed; Group 1 - irradiated with a Neutral Beam derived from a GCIB accelerated with a V_{Acc} of 30kV. The Group 1 irradiated beam energy dose was equivalent to that of a 30kV accelerated, 5 x 10¹⁴ gas cluster ion per cm² dose (energy equivalence determined by beam thermal energy flux sensor); Group 2 - irradiated with a Neutral Beam derived from a GCIB accelerated with a V_{Acc} of 30kV. The Group 2 irradiated beam energy dose was equivalent to that of a 30kV accelerated, 1 x 10¹⁴ gas cluster ion per cm² dose (energy equivalence determined by beam thermal energy flux sensor); and Group 3 - irradiated with a Neutral Beam derived from a GCIB accelerated with a V_{Acc} of 25kV. The Group 3 irradiated beam energy dose was equivalent to that of a 25kV accelerated, 5 x 10¹⁴ gas cluster ion per cm² dose (energy equivalence determined by beam thermal energy flux sensor).

**TABLE 1**

| Group [Dose] {V_{Acc}} | Control | | | Group 1 [5 x 10¹⁴] {30 kV} | | | Group 2 [1 x 10¹⁴] {30 kV} | | | Group 3 [5 x 10¹⁴] {25 kV} | | |
|---|---|---|---|---|---|---|---|---|---|---|---|---|
| Coupon # | Start Load (µg) | Elution Loss (µg) | Elution Loss % | Start Load (µg) | Elution Loss (µg) | Elution Loss % | Start Load (µg) | Elution Loss (µg) | Loss % | Start Load (µg) | Elution Loss (µg) | Elution Loss % |
| 1 | 83 | 60 | 72 | 88 | 4 | 5 | 93 | 10 | 11 | 88 | - | 0 |
| 2 | 87 | 55 | 63 | 100 | 7 | 7 | 102 | 16 | 16 | 82 | 5 | 6 |
| 3 | 88 | 61 | 69 | 83 | 2 | 2 | 81 | 35 | 43 | 93 | 1 | 1 |
| 4 | 96 | 72 | 75 | - | - | - | 93 | 7 | 8 | 84 | 3 | 4 |
| Mean | 89 | 62 | 70 | 90 | 4 | 5 | 92 | 17 | 19 | 87 | 2 | 3 |
| σ | 5 | 7 | | 9 | 3 | | 9 | 13 | | 5 | 2 | |
| p value | | | | 0.00048 | | | 0.014 | | | 0.00003 | | |

Table 1 shows that for every case of Neutral Beam irradiation (Groups 1 through 3), the drug lost during a 4.5-hour elution into human plasma was much lower than for the un-irradiated Control Group. This indicates that the Neutral Beam irradiation results in better drug adhesion and/or reduced elution rate as compared to the un-irradiated drug. The p values (heterogeneous unpaired T-test) indicate that for each of the Neutral Beam irradiated Groups 1 through 3, relative to the Control Group, the difference in the drug retention following elution in human plasma was statistically significant.

To confirm that complex molecules such as proteins are not destroyed by GCIB or Neutral Beam irradiation when layers containing them are treated to form barrier layers for controlling their release, evaluations were made using the bone morphogenic protein BMP2 in combination with GCIB and Neutral Beam irradiation and assays to determine protein degradation and protein loss.

Titanium foils were cut to 1x1 cm and cleaned in 70% isopropanol for 30 minutes followed by tw0 10 minute washes in double distilled water. Recombinant human protein BMP2 (rhBMP2) supplied from R&D Systems, Inc., 614 McKinley Place NE, Minneapolis, MN 55413, USA was reconstituted in 4mM HCl at 100ng/µl. 18 Ti foils were spotted with 10µl BMP2 (1µg) and allowed to air dry for 1 hour. The 18 foils were divided into the following groups: 1) GCIB irradiated, 5 x 10¹⁴ ions/cm² dose, low acceleration potential (n=3 pieces); 2) GCIB irradiated, 5 x 10¹⁴ ions/cm² dose, high acceleration potential (n=3 pieces); 3) Neutral Beam irradiated, 5 x 10¹⁴ ions/cm² dose (thermal energy dose equivalent), low acceleration potential (n=3 pieces); 4) Neutral Beam irradiated, 5 x 10¹⁴ ions/cm² dose (thermal energy dose equivalent), high acceleration potential (n=3 pieces). For each condition listed, n=1 piece was used for degradation silver stain assay and n=2 pieces were used for Enzyme-Linked ImmunoSorbent Assay (ELISA) tests. High acceleration potential was 30 kV, and low acceleration potential was 10 kV in each case.

Degradation assay: 1x cell lysis buffer from Cell Signaling Technology (#9803) with HALT protease inhibitor (Thermo Scientific #87786) was prepared according to manufacturer's instructions; 15µl was placed on the Ti foil and pipetted up and down 5 times to extract protein from surface and placed in Eppendorf micro tubes. As a control, pure protein, 5µl rhBMP2 (500ng) was placed in a separate tube. To each tube, was added 15µl Laemmli sample buffer (Bio-Rad #161-0737) with 2-mercaptoethanol (Bio-Rad #161-0710) according to manufacturer's instructions, boiled 5 min, iced 2 min. Samples were loaded on 18% sodium dodecyl sulfate polyacrylamide electrophoresis gel and driven at 75V until dye front was near bottom (approximately 2 hours). The gel was removed and stained with silver stain (Bio-Rad #161-0449) per manufacturer's instructions.

Degradation assay results: GCIB and Neutral Beam at both high and low energies appeared to have no degredative effects on BMP2. Proteins extracted from the Ti foils appeared to match pure BMP2 protein in electrophoresis response. No degradation products were visible below BMP2 size (15-16 kDa). Any protein modified in the formation of the resulting barrier layer does not appear to be eluted as degradation product.

ELISA assay: An R&D Systems, Inc. Human BMP2 Quantikine ELISA kit was used to assay BMP2. To each Ti foil (see above), was added 500µl 1x lysis buffer and was allowed to reside on the foil for 10 minutes followed by pipetting up and down 5 times to extract protein from surface and was placed in micro tubes. Manufacturer's instructions for the ELISA kit (R&D Systems #DBP200) were followed to perform the assay. Briefly, initial concentration was diluted 500 fold to fall within the range of the kit using 1x calibrator diluent. Duplicates from each sample were used and an initial n=2 per foil sample resulting in 4 individual assay reads per sample. A standard curve using BMP2 was generated and both standards and unknown concentrations of samples were placed on wells pre-conjugated with BMP2 monoclonal antibody against the full length BMP2 protein. Following binding to the wells, a conjugated anti-BMP2 bound to horseradish peroxidase was added to create a sandwich ELISA. A colorimetric substrate was added, and following color development, the color was measured on a colorimetric plate reader at 450nm wavelength with background corrected at 570nm. Concentrations of the samples were calculated from the standard curve.

ELISA assay results: Results revealed that Control samples, ie. samples not irradiated with GCIB or Neutral Beam recovered fully at 1.00 ± 0.02 µg BMP2. GCIB low energy resulted in 0.80 ± 0.03 µg BMP2. GCIB high energy recovery was at 0.71 ± 0.10 µg BMP2. Neutral Beam low energy resulted in 0.70 ± 0.14 µg BMP2 and Neutral Beam high energy recovery was 0.70 ± 0.02 µg BMP2. As the antibody against the BMP2 is against the full length protein, the amount recovered represents the amount of active BMP2 present. It is believed that the amounts lost may be in part due to vacuum sublimation in the GCIB/Neutral Beam tool, the actual GCIB or Neutral Beam irradiation process, and loss due to the resulting modification of BMP2 to form the resulting barrier layer, which is not active protein.

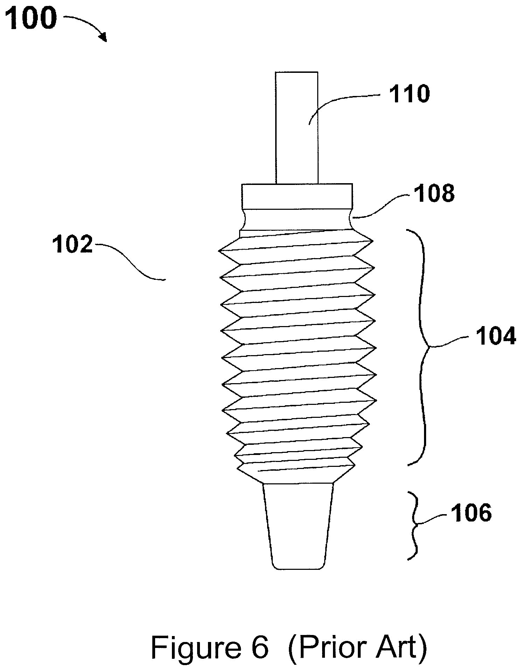

Reference is now made to Figure 6, which shows a view 100 of a prior art bone-implantable medical device in the form of a prior art dental implant 102. The prior art dental implant 102 is used for insertion into and implantation into a hole in a jawbone to form a basis for attaching a dental prosthesis, such as a prosthetic tooth or a dental restoration, for example. Drilling or other surgical techniques are typically employed to form the jawbone hole. The prior art dental implant 102 has a post 110 for attachment of a dental prosthesis (not shown). It has an implantable portion consisting of a threaded portion 104 and an unthreaded portion 106. A neck 108 connects the implantable portion with the post 110. The prior art dental implant 102 may be a single piece, or a composite of two or more pieces joined by any of a variety of known fastening techniques. In general the materials of the outer surfaces of the implantable portion are formed from biocompatible materials, often metal, oxide, or ceramic, preferably titanium with a titania (native or otherwise) surface or zirconia. Prior art dental implants are manufactured according to numerous different configurations, but in general they all have an implantable portion intended to be implanted into a hole or otherwise attached to a bone. The prior art dental implant 102 has a threaded portion 104 intended to intimately engage a hole in a bone, where if the implant is successful, it eventually becomes integrated with the bone by regrowth of new bone material.

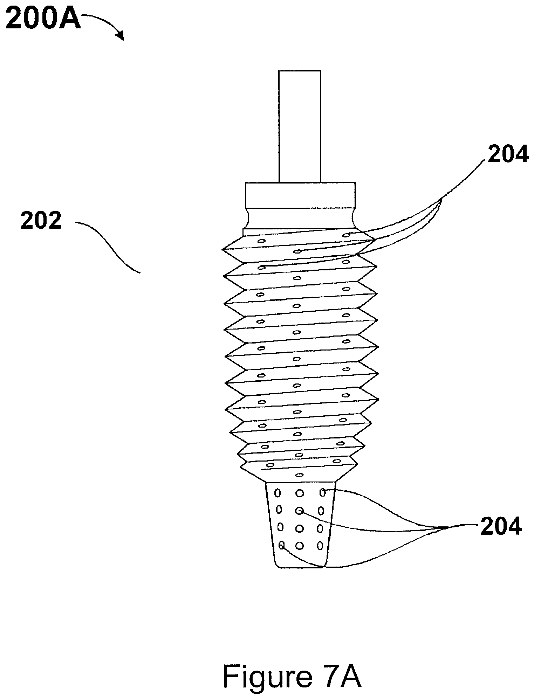

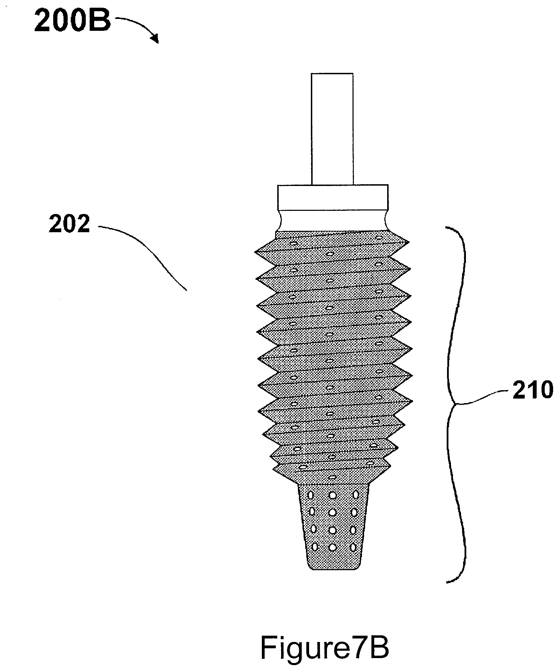

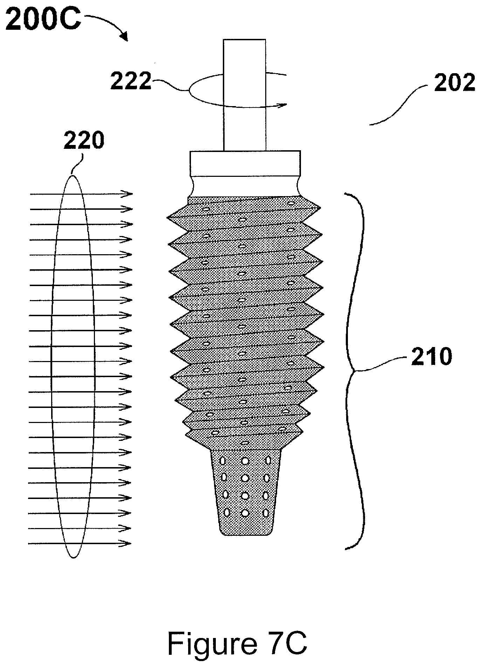

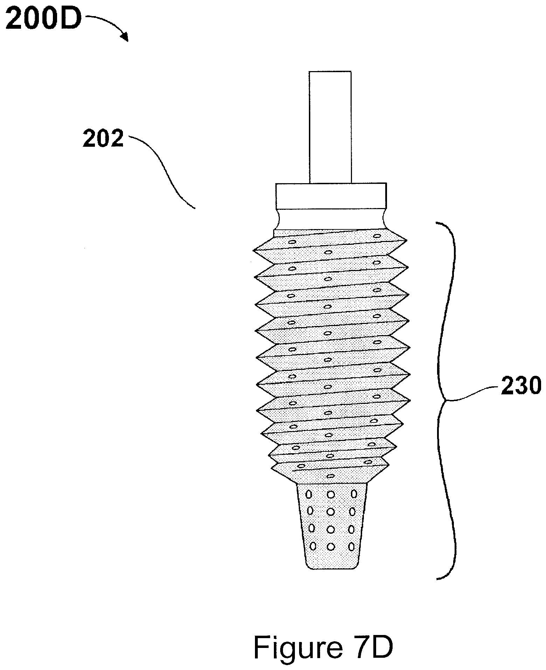

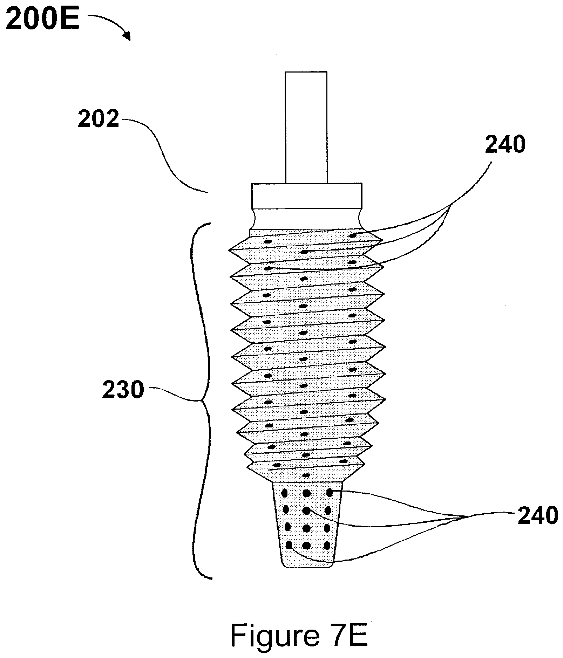

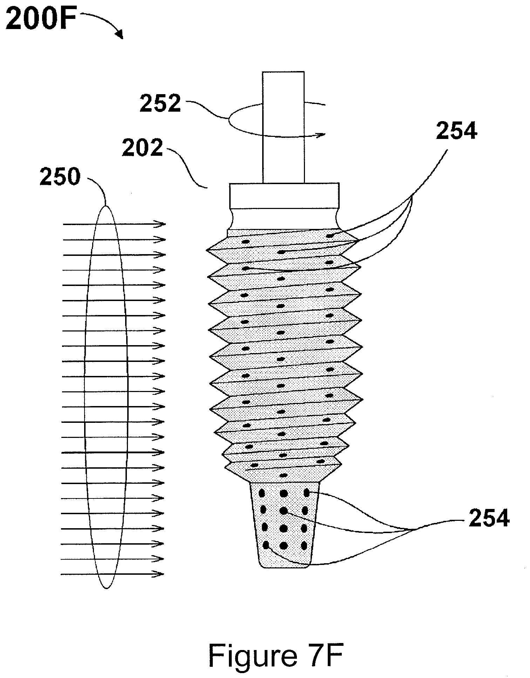

Figure 7A is a view 200A of a bone-implantable medical device in the form of a dental implant 202 as may be employed in an embodiment of the present invention. Dental implant 202 is an improved form of the prior art dental implant 102 (as shown in Figure 6). Referring again to Figure 7A, the implantable portion of the dental implant 202 preferably has a titania surface and has a multiplicity of holes (examples indicated by 204, not all holes labeled). Although shown in a particular pattern for explaining the invention, the particular pattern shown is not essential to the invention and it is understood that many and varied patterns can be employed in various embodiments of the invention. The relative sizes of the dental implant 202 and the holes 204 are not necessarily shown to scale. The holes may have a wide range of sizes and shapes. The holes 204 can be formed by a variety of techniques, but the methods of laser machining and focused ion beam machining are preferable because they can be controlled with great precision and can produce small, deep holes. The holes 204 may be, for example from about 50 micrometers to about 500 micrometers in diameter (or width) and may have an aspect ratio (diameter or width to depth) of about 0.5 to about 10 or even more. The holes 204 may be circular as indicated in Figure 7 or in the form of grooves, trenches, other shapes, or combinations. The holes 204 may be of a variety of different diameters (or widths) and aspect ratios and (if grooves or trenches) lengths and shapes, so as to have differing volumes. The holes 204, empty at this process step, are provided for holding one or more therapeutic agents as for example BMP and/or antibiotics, anti-inflammatory agents, etc.

Figure 7B is a view 200B of the dental implant 202, after additional processing according to an embodiment of the invention. A portion of the surface of the dental implant is shown as coated with an osteoinductive agent, for example HA. The coated surface portion 210 (indicated in the figure by coarse stippling), in this case corresponds with the surface of the implantable portion of the dental implant 202, but could alternatively be one or more smaller regions of the implantable portion of the dental implant 202, with other regions uncoated. The osteoinductive agent coating may be applied by any of several methods, including for examples, spraying or suspension of ultra-fine particles, precipitation from solution, dipping, electrostatic deposition, ultrasonic spraying, plasma spraying, and sputter coating. During coating, a conventional masking scheme may be employed to limit deposition to a selected location or locations. A coating thickness of from about 0.01 to about 5 micrometers may preferably be utilized. At this step of the process, the coating of osteoinductive agent is still susceptible to the problems described above - it can be abraded away or otherwise undesirably removed during the mechanical stresses of implantation into bone.

Figure 7C shows a view 200C of the dental implant 202 after a portion of the surface has been coated with osteoinductive agent. An ion beam, preferably GCIB 220 is now employed to irradiate the coated surface portion 210 of the dental implant 202 to form a thin osteoinductive agent-infused layer in the preferably titania or zirconia surface of the implantable portion of the dental implant 202 wherever the coated surface portion 210 previously existed. However, if for any reason it is not desired to form an osteoinductive agent-infused layer at any portion of the osteoinductive agent-coated surface, a conventional masking scheme or controlled direction of the GCIB 220 may be employed to limit irradiation to selected locations. Although the infused layer has been described, for example, as an HA-infused layer it will be readily understood, that if a different osteoinductive agent is used to form the coated surface portion 210, then the infused layer will be an infused layer of the different agent. In infusing the osteoinductive agent into the surface of the dental implant 202, a GCIB 220 comprising preferably argon cluster ions or oxygen cluster ions may be employed. The GCIB 220 may be accelerated with an accelerating potential of from 5kV to70kV or more. The coating may be exposed to a GCIB dose of at least about 1 x 10¹⁴ gas cluster ions per square centimeter. The GCIB irradiation step produces an osteoinductive agent-infused layer within the immediate surface of the titania or zirconia that is on the order of from about 1 to about 10 nanometers thick. While performing the ion beam irradiation, it is preferable to rotate the dental implant 202 about its axis with a rotary motion 222 during irradiation to assure that the desired ion dose is achieved on the entire coated surface portion 210. US Pat. 6,676,989C1 issued to Kirkpatrick et al. teaches a GCIB processing system having a holder and manipulator suited for rotary processing tubular or cylindrical workpieces such as vascular stents and with routine adaptation, that system is also capable of the rotary irradiation required for this invention. In certain cases it may be desirable to infuse larger quantities of osteoinductive agent than can conveniently be done in a single coating and GCIB irradiation. In such cases, it is within the scope of the invention to repeat (one or more times) the steps of (a) coating the desired areas of the dental implant 202 with osteoinductive agent, and (b) irradiating the coated areas with GCIB to infuse the additional osteoinductive agent (using the techniques described herein in the descriptions of FIGS. 2B and 2C.

Figure 7D shows a view 200D of the dental implant 202 following the HA infusion step. The osteoinductive agent-coated portion has been fully converted to an osteoinductive agent-infused surface region 230 according to an embodiment of the invention.

Figure 7E shows a view 200E of a dental implant 202, having an osteoinductive agent infused surface region. At this step, the holes 204 (as shown in Figure2A) are now loaded with a therapeutic material, forming loaded holes 240 (again referring to Figure 7E). The loading of the therapeutic material may be done by any of numerous methods, including spraying, dipping, wiping, electrostatic deposition, ultrasonic spraying, vapor deposition, or by discrete droplet-on-demand fluid jetting technology. When spraying, dipping, wiping, electrostatic deposition, ultrasonic spraying, vapor deposition, or similar techniques are employed, a conventional masking scheme may be beneficially employed to limit deposition to a hole or to several or all of the holes in the dental implant 202. For liquids and solutions, discrete droplet-on-demand fluid-jetting is a preferred deposition method because it provides the ability to introduce precise volumes of liquid materials or solutions into precisely programmable locations. Discrete droplet-on-demand fluid jetting may be accomplished using commercially available fluid-jet print head jetting devices as are available (for example, not limitation) from MicroFab Technologies, Inc., of Plano, Texas. When the therapeutic material is a liquid, liquid suspension, or a solution, it is preferably dried or otherwise hardened before proceeding to the next step. The drying or hardening step may include baking, low temperature baking, or vacuum evaporation, as examples.

Figure 7F is a view 200F showing an ion irradiation step in the formation of a thin barrier layer in the surface of the therapeutic agent loaded in the holes in the dental implant 202. An ion beam, preferably GCIB 250 is now employed to irradiate the surface of the therapeutic agent in the loaded holes 240 (see Figure 7E) in the dental implant 202 to form a thin barrier layer at the surface to the therapeutic agent, forming loaded holes with thin barrier layers 254 at the exposed surface. The GCIB 250 forms a thin barrier layer at the surface of the therapeutic agent in the holes by modification of a thin upper region of the therapeutic agent. The thin barrier layer consists of therapeutic agent modified so as to densify, carbonize or partially carbonize, denature, cross-link, or polymerize molecules of the therapeutic material in the thin uppermost layer of the therapeutic material. The thin barrier layer may have a thickness on the order of about 10 nanometers or even less. Additional details on the thin barrier layer and the process of its formation are described hereinafter in the discussion of Figures 3C and 3D.

Figures 3A, 3B, 3C, and 3D show detail of the steps for loading holes in a bone-implantable medical device with a therapeutic agent, and forming a thin barrier layer thereon for controlling retention and elution of the therapeutic agent by using GCIB irradiation.

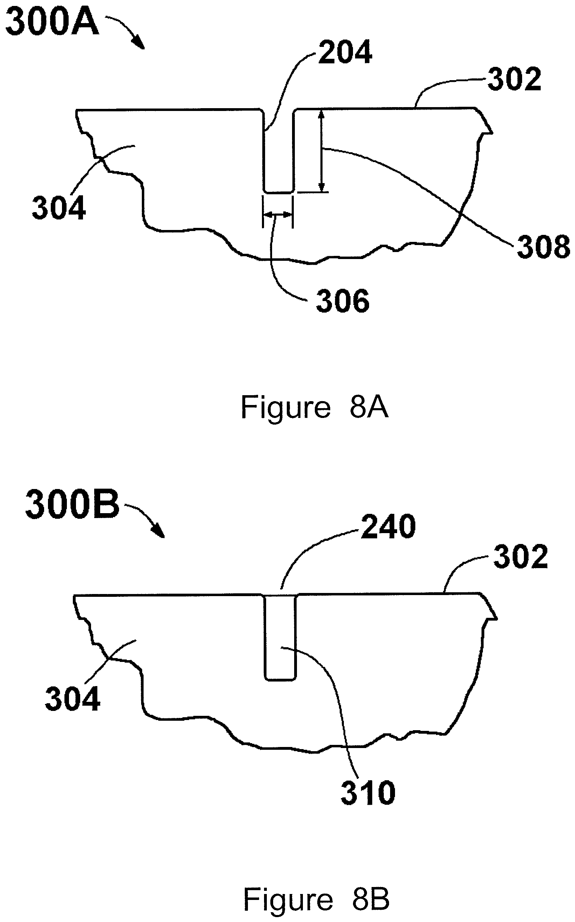

Figure 8A shows a view 300A of a portion 304 of a surface 302 of a dental implant 202 (as shown in at the stage indicated in Figure 7D, i.e., having an osteoinductive agent-infused surface region according to the invention), wherein the surface 302 represents a portion of the osteoinductive agent-infused surface region. Again referring to Figure 8A, the portion 304 of the surface 302 of the dental implant 202 has a hole 204 having a diameter 306 and a depth 308. In this instance the hole 204 is intended to represent a substantially cylindrical hole, but as previously explained, other hole configurations are expected within the scope of the invention and the cylindrical nature of the hole is not intended to be limiting, but rather for clear explanation of the invention. The hole 204 is at a stage of readiness for loading with a therapeutic agent.

Figure 8B shows a view 300B of a portion 304 of a surface 302 of a dental implant 202 with an osteoinductive agent-infused surface region. In this stage, the hole 204 has been loaded with a therapeutic agent 310, forming a loaded hole 240, corresponding to the loaded hole of Figure 7E.

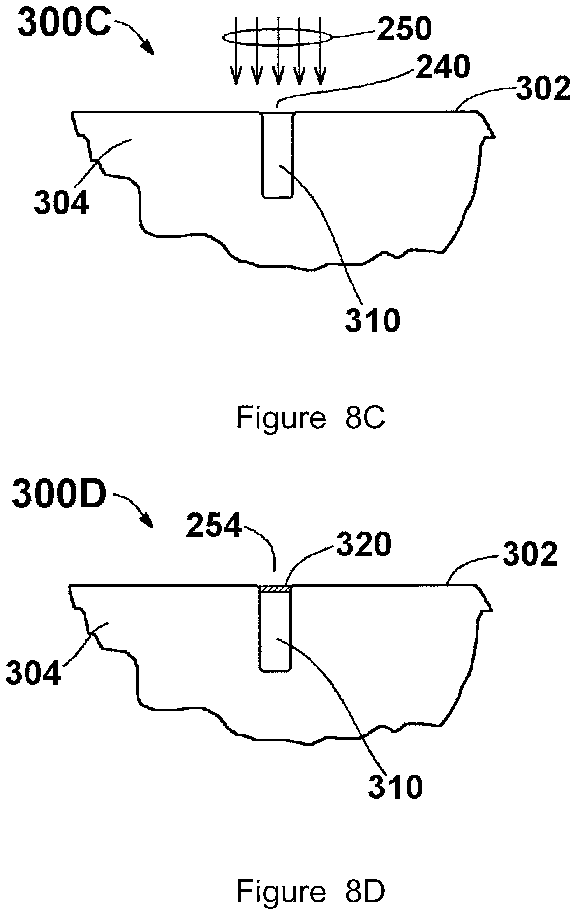

Figure 8C shows a view 300C of a portion 304 of a surface 302 of a dental implant 202 with an osteoinductive agent-infused surface region and a loaded hole 240 loaded with therapeutic agent 310. An ion beam, preferably GCIB 250 is directed at the surface of the therapeutic agent 310 for the purpose of modifying the uppermost part of the surface of the therapeutic agent 310 to form a barrier layer. The therapeutic agent 310 is irradiated by the GCIB 250 modify of a thin upper region of the surface of the therapeutic agent 310. In modifying the surface, a GCIB 250 comprising preferably argon cluster ions or cluster ions of another inert gas may be employed. The GCIB 250 is may be accelerated with an accelerating potential of from 5kV to50kV or more. The upper surface of the therapeutic agent 310 is may be exposed to a GCIB dose of at least about 1x10¹³ gas cluster ions per square centimeter.

Figure 8D shows a view 300D of a portion 304 of a surface 302 of a dental implant 202 with an osteoinductive agent-infused surface region and a hole 254 that is loaded with a therapeutic agent 310 upon which has been formed a thin barrier layer 320 by ion irradiation of the uppermost surface of the therapeutic agent 310. The thin barrier layer 320 consists of therapeutic agent 310 modified so as to densify, carbonize or partially carbonize, denature, cross-link, or polymerize molecules of the therapeutic agent in the thin uppermost layer of the therapeutic agent 310. The thin barrier layer 320 may have a thickness on the order of about 10 nanometers or even less. By selecting the dose and/or accelerating potential of the GCIB 250 (FIGS. 2F and 3C), the characteristics of the thin barrier layer 320 may be adjusted to permit control of the elution rate and/or the rate of inward diffusion of water and/or other biological fluids when the dental implant 202 is implanted in bone. In general, increasing acceleration potential increases the thickness of the thin barrier layer that is formed, and modifying the GCIB dose changes the nature of the thin barrier layer by changing the degree of cross linking, densification, carbonization, denaturization, and/or polymerization that results. This provides means to control the rate at which the therapeutic agent 310 will subsequently release or elute through the barrier and/or the rate at which water and/or biological fluids my diffuse into the drug from outside the dental implant 202.

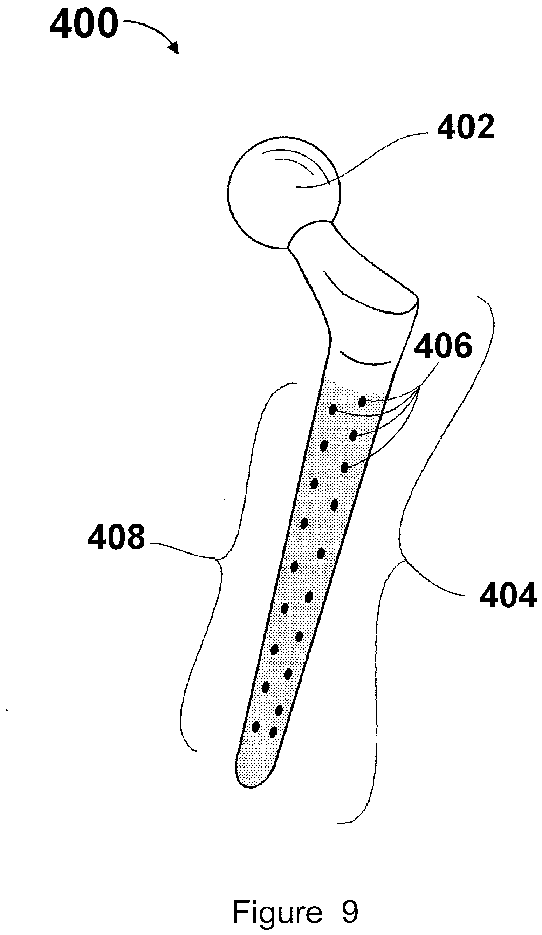

Figure 9 shows a bone-implantable medical device in the form of an artificial hip joint prosthesis 400 for replacement of a femoral ball. The prosthesis 400 has a ball 402 for replacement of the ball portion of the natural joint and has a stem 404 for insertion into and for integration with the femur. According to the invention, a portion 408 of the surface of the stem 404 has been osteoinductive agent-infused by osteoinductive agent-coating followed by ion irradiation (preferably GCIB irradiation) and has a pattern of holes 406 that are loaded with a therapeutic agent and which have been ion irradiated (preferably GCIB irradiated) to form thin barrier layers for control of elution rate of the therapeutic agent.

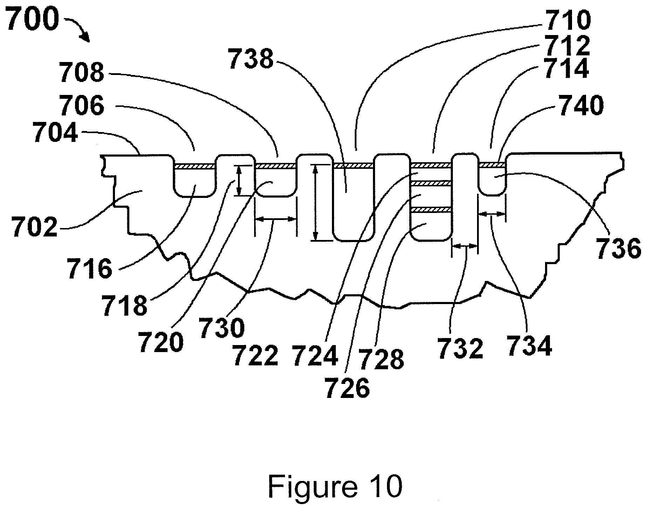

Figure 10 (not part of the invention) shows a cross sectional view 700 of the surface 704 of a portion 702 of a bone-implantable medical device having a variety of therapeutic agent loaded holes 706, 708, 710, 712, and 714 shown to point out the diversity and flexibility of the invention. The bone-implantable medical device could, for example, be any of a dental implant, a bone screw, an artificial joint prosthesis, or any other bone-implantable medical device. The holes all have thin barrier layers 740 formed according to the invention on one or more layers of therapeutic agent in each hole. For simplicity, not all of the thin barrier layers in Figure 10 are labeled with reference numerals, but hole 714 is shown containing a first therapeutic agent 736 covered with a thin barrier layer 740 (only thin barrier layer 740 in hole 714 is labeled with a reference numeral, but each cross-hatched region in Figure 10 indicates a thin barrier layer, and all will hereinafter be referred to by the exemplary reference numeral 740). Hole 706 contains a second therapeutic agent 716 covered with a thin barrier layer 740. Hole 708 contains a third therapeutic agent 720 covered with a thin barrier layer 740. Hole 710 contains a fourth therapeutic agent 738 covered with a thin barrier layer 740. Hole 712 contains fifth, sixth, and seventh therapeutic agents 728, 726, and 724, each respectively covered with a thin barrier layer 740. Each of the respective therapeutic agents 716, 720, 724, 726, 728, 736, and 738 may be selected to be a different therapeutic agent material or may be the same therapeutic agent materials in various combinations of different or same. Each of the thin barrier layers 740 may have the same or different properties for controlling elution or release rate and/or for controlling the rate of inward diffusion of water or other biological fluids according to ion beam processing (preferably GCIB processing) principles discussed herein above. Holes 706 and 708 have the same widths and fill depth 718, and thus hold the same volume of therapeutic agents, but the therapeutic agents 716 and 720 may be different therapeutic agents for different therapeutic modes. The thin barrier layers 740 corresponding respectively to holes 706 and 708 may have either same or differing properties for providing same or different elution, release, or inward diffusion rates for the therapeutic agents contained in holes 706 and 708. Holes 708 and 710 have the same widths, but differing fill depths, 718 and 722 respectively, thus containing differing therapeutic agent loads corresponding to differing doses. The thin barrier layers 740 corresponding respectively to holes 708 and 710 may have either same or differing properties for providing same or different elution, release, or inward diffusion rates for the therapeutic agents contained in holes 708 and 710. Holes 710 and 712 have the same widths 730, and have the same fill depths 722, thus containing the same total therapeutic agent loads, but hole 710 is filled with a single layer of therapeutic agent 738, while hole 712 is filled with multiple layers of therapeutic agent 724, 726, and 728, which may each be the same or different volumes of therapeutic agent representing the same or different doses and furthermore may each be different therapeutic agent materials for different therapeutic modes. Each of the thin barrier layers 740 for holes 710 and 712 may have the same or different properties for providing same or different elution, release, or inward diffusion rates for the therapeutic agents contained in the holes. Holes 708 and 714 have the same fill depths 718, but have different widths and thus contain different volumes and doses of therapeutic agents 720 and 736. The thin barrier layers 740 corresponding respectively to holes 708 and 714 may have either same or differing properties for providing same or different elution, release, or inward diffusion rates for the therapeutic agents contained in holes 708 and 714. The overall hole (size, shape, and location) pattern on the surface 704 of the implantable medical device and the spacing between holes 732 may additionally be selected to control distribution of therapeutic agent dose across the surface of the implantable medical device. Thus there are many flexible options in the application of the invention for controlling the types and doses and dose distributions and release sequences and release rates of therapeutic agents contained in the bone-implantable medical devices of the invention.

Although the invention has been described with respect to formation of exemplary HA-infused layers, it is recognized that other osteoinductive agents can equally well be employed in forming the infused layers within the scope of the invention. Although the invention has particularly been described in terms of application to titanium (with titania surface) and zirconia dental implants, it is recognized that the scope of the invention includes bone-implantable medical devices constructed of a wide variety of other materials such as, for example polyether
ether ketone (PEEK). In the case of electrically insulating materials such as PEEK, the accelerated Neutral Beam has the advantage of reduced damage in the insulating substrate as compared to a GCIB or other ion beam.

## Claims

1. A method of modifying a surface of a bone-implantable medical device comprising the steps of:
coating at least a first portion of the surface of the medical device with an osteoinductive agent to form a coated surface region; and
first irradiating at least a portion of the coated surface region with a first accelerated Neutral Beam.

2. The method of claim 1, wherein the first irradiating step forms a shallow surface and subsurface layer comprising embedded molecules and/or dissociation products of the osteoinductive agent.

3. The method of claim 1, wherein the first accelerated Neutral Beam is derived from a first gas cluster ion beam and further wherein the shallow surface and subsurface layer is an infused surface layer.

4. The method of claim 1, wherein the osteoinductive agent comprises, separately or in combination, any of the materials from the group consisting of: a nutrient material, tricalcium phosphate, hydroxyapatite, Bioglass 45S5, Bioglass 58S, a bone growth-stimulating agent, a growth factor, a cytokine, a TGF-[beta] protein, a BMP, a GPI-anchored signaling protein, an RGM, and a growth regulatory protein.

5. The method of claim 1, wherein the surface of the bone-implantable medical device comprises, separately or in combination, any of a metal, an oxide, or a ceramic and, optionally, wherein the surface comprises titanium, titania, or zirconia.

6. The method of claim 1, further comprising the steps, prior to the coating step either:
(a) forming a second ion beam that is a gas cluster ion beam; and second irradiating at least a second portion of the surface of the medical device to clean the at least a second portion of the surface; or
(b) forming a second accelerated Neutral Beam; and second irradiating at least a second portion of the surface of the medical device to clean the at least a second portion of the surface.

7. The method of claim 1, wherein the first irradiating step further comprises
(a) employing a mask to control the at least a portion of the coated surface region that is irradiated; or
(b) positioning the medical device with respect to the first accelerated Neutral Beam to control the at least a portion of the coated surface region that is irradiated.

8. The method of claim 1, further comprising the steps of:
forming one or more holes in the surface of the medical device;
first loading at least one of the one or more holes with a first therapeutic agent; and
third irradiating an exposed surface of the first therapeutic agent in at least one loaded hole with a third accelerated Neutral Beam to form a barrier layer at the exposed surface.

9. The method of claim 8, wherein the third accelerated Neutral Beam is derived from an accelerated gas cluster ion beam.

10. The method of claim 8, wherein the barrier layer controls
(a) an elution rate of therapeutic agent; or
(b) a rate of inward diffusion of a fluid into the hole.

11. The method of claim 8, wherein the one or more holes are disposed on the surface in a predetermined pattern to distribute the first therapeutic agent on the surface according to a predetermined distribution plan.

12. The method of claim 8, wherein at least one of the one or more holes is loaded with
(a) a second therapeutic agent different from the first therapeutic agent; or
(b) a first quantity of the first therapeutic agent that differs from a second quantity of the first therapeutic agent loaded in at least another of the one or more holes.

13. The method of claim 8, wherein the first loading step does not completely fill the at least one hole, and following the first irradiating step further comprising the steps of:
second loading the at least one incompletely filled hole with a second therapeutic agent overlying the first barrier layer; and
fourth irradiating an exposed surface of the second therapeutic agent in at least one second loaded hole with a fourth accelerated Neutral Beam to form a second barrier layer at the exposed surface in the at least one loaded hole; and, optionally,
wherein the first barrier layer and the second barrier layer have different properties for controlling elution rate of the first and second therapeutic agents.

14. The method of claim 13, wherein the third accelerated Neutral Beam is derived from an accelerated gas cluster ion beam and further, optionally, wherein the fourth accelerated Neutral Beam is derived from an accelerated gas cluster ion beam.

15. A bone-implantable medical device having a surface, wherein at least a portion of the surface comprises a shallow Neutral Beam infused surface and subsurface layer of 10 nanometres or less comprising molecules and/or atoms or larger fragments of molecules of an osteoinductive agent embedded into the at least a portion of the surface.

16. The medical device of claim 15, further comprising:
one or more holes containing a therapeutic agent in the surface of the medical device; and
at least one or more barrier layers at one or more surfaces of the therapeutic agent contained in the one or more holes; wherein
the at least one or more barrier layers control at least one elution rate of therapeutic agent.

17. The medical device of claim 15, wherein the surface of the medical device comprises titanium, titania, or zirconia.

## Patentansprüche

1. Verfahren zum Modifizieren einer Fläche einer knochenimplantierbaren medizinischen Vorrichtung, das die folgenden Schritte umfasst:
Beschichten mindestens eines ersten Abschnitts der Fläche der medizinischen Vorrichtung mit einem knocheninduzierenden Mittel, um eine beschichtete Flächenregion zu bilden; und
erstes Bestrahlen mindestens eines Abschnitts der beschichteten Flächenregion mit einem ersten beschleunigten Neutralstrahl.

2. Verfahren nach Anspruch 1, wobei der erste Bestrahlungsschritt eine flache Fläche und eine unter der Fläche liegende Schicht bildet, die eingebettete Moleküle und/oder Spaltprodukte des knocheninduzierenden Mittels umfasst.

3. Verfahren nach Anspruch 1, wobei der erste beschleunigte Neutralstrahl von einem ersten Gasclusterionenstrahl abgeleitet ist und ferner wobei die flache Fläche und die unter der Fläche liegende Schicht eine infundierte Flächenschicht sind.

4. Verfahren nach Anspruch 1, wobei das knocheninduzierende Mittel separat oder in Kombination beliebige der Materialien aus der Gruppe umfasst, die aus Folgendem besteht: einem Nährstoff, Tricalciumphosphat, Hydroxyapatit, Bioglas 45S5, Bioglas 58S, einem knochenwachstumstimulierenden Mittel, einem Wachstumsfaktor, einem Zytokin, einem TGF-[beta]-Protein, einem BMP, einem GPI-verankerten Signalprotein, einem RGM und einem wachstumsregulatorischen Protein.

5. Verfahren nach Anspruch 1, wobei die Fläche der knochenimplantierbaren medizinischen Vorrichtung separat oder in Kombination beliebige von einem Metall, einem Oxid oder einer Keramik umfasst, und wobei die Fläche gegebenenfalls Titan, Titandioxid oder Zirkoniumdioxid umfasst.

6. Verfahren nach Anspruch 1, ferner einen der folgenden Schritte vor dem Beschichtungsschritt umfassend:
(a) Bilden eines zweiten Ionenstrahls, der ein Gasclusterionenstrahl ist; und zweites Bestrahlen mindestens eines zweiten Abschnitts der Fläche der medizinischen Vorrichtung, um den mindestens einen zweiten Abschnitt der Fläche zu reinigen; oder
(b) Bilden eines zweiten beschleunigten Neutralstrahls; und zweites Bestrahlen mindestens eines zweiten Abschnitts der Fläche der medizinischen Vorrichtung, um den mindestens einen zweiten Abschnitt der Fläche zu reinigen.

7. Verfahren nach Anspruch 1, wobei der erste Bestrahlungsschritt ferner Folgendes umfasst:
(a) Einsetzen einer Maske, um den mindestens einen Abschnitt der beschichteten Flächenregion, der bestrahlt wird, zu steuern; oder
(b) Positionieren der medizinischen Vorrichtung in Bezug auf den ersten beschleunigten Neutralstrahl, um den mindestens einen Abschnitt der beschichteten Flächenregion, die bestrahlt wird, zu steuern.

8. Verfahren nach Anspruch 1, ferner die folgenden Schritte umfassend:
Bilden eines oder mehrerer Löcher in der Fläche der medizinischen Vorrichtung;
erstes Beladen mindestens eines des einen oder der mehreren Löcher mit einem ersten therapeutischen Mittel; und
drittes Bestrahlen einer freiliegenden Fläche des ersten therapeutischen Mittels in mindestens einem beladenen Loch mit einem dritten beschleunigten Neutralstrahl, um eine Barriereschicht an der freiliegenden Fläche zu bilden.

9. Verfahren nach Anspruch 8, wobei der dritte beschleunigte Neutralstrahl von einem beschleunigten Gasclusterionenstrahl abgeleitet ist.

10. Verfahren nach Anspruch 8, wobei die Barriereschicht Folgendes steuert:
(a) eine Elutionsrate des therapeutischen Mittels; oder
(b) eine Rate von Eindiffundieren eines Fluids in das Loch.

11. Verfahren nach Anspruch 8, wobei das eine oder die mehreren Löcher auf der Fläche in einem vorbestimmten Muster angeordnet sind, um das erste therapeutische Mittel auf der Fläche gemäß einem vorbestimmten Verteilungsplan zu verteilen.

12. Verfahren nach Anspruch 8, wobei mindestens eines des einen oder der mehreren Löcher mit Folgendem beladen wird:
(a) einem zweiten therapeutischen Mittel, das sich von dem ersten therapeutischen Mittel unterscheidet; oder
(b) eine erste Menge des ersten therapeutischen Mittels, die sich von einer zweiten Menge des ersten therapeutischen Mittels unterscheidet, das in mindestens einem anderen des einen oder der mehreren Löcher geladen ist.

13. Verfahren nach Anspruch 8, wobei der erste Beladungsschritt das mindestens eine Loch nicht vollständig füllt, und wobei das Verfahren nach dem ersten Bestrahlungsschritt ferner die folgenden Schritte umfasst:
zweites Beladen des mindestens einen unvollständig gefüllten Lochs mit einem zweiten therapeutischen Mittel, das über der ersten Barriereschicht liegt; und
viertes Bestrahlen einer freiliegenden Fläche des zweiten therapeutischen Mittels in mindestens einem zweiten beladenen Loch mit einem vierten beschleunigten Neutralstrahl, um eine zweite Barriereschicht an der freiliegenden Fläche in dem mindestens einem beladenen Loch zu bilden; und gegebenenfalls wobei die erste Barriereschicht und die zweite Barriereschicht verschiedene Eigenschaften zur Elutionsratensteuerung des ersten und des zweiten therapeutischen Mittels aufweisen.

14. Verfahren nach Anspruch 13, wobei der dritte beschleunigte Neutralstrahl von einem beschleunigten Gasclusterionenstrahl abgeleitet ist und ferner wobei der vierte beschleunigte Neutralstrahl gegebenenfalls von einem beschleunigten Gasclusterionenstrahl abgeleitet ist.

15. Knochenimplantierbare medizinische Vorrichtung, die eine Fläche aufweist, wobei mindestens ein Abschnitt der Fläche eine flache neutralstrahlinfundierte Fläche und eine unter der Fläche liegende Schicht von 10 nm oder weniger umfasst, die Moleküle und/oder Atome oder größere Fragmente von Molekülen eines knocheninduzierenden Mittels umfasst, die in mindestens einem Abschnitt der Fläche eingebettet sind.

16. Medizinische Vorrichtung nach Anspruch 15, ferner Folgendes umfassend:
ein oder mehrere Löcher, die ein therapeutisches Mittel in der Fläche der medizinischen Vorrichtung enthalten; und
mindestens eine oder mehrere Barriereschichten an einer oder mehreren Flächen des therapeutischen Mittels, das in dem einem oder den mehreren Löchern enthalten ist; wobei
die mindestens eine oder mehreren Barriereschichten mindestens eine Elutionsrate des therapeutischen Mittels steuern.

17. Medizinische Vorrichtung nach Anspruch 15, wobei die Fläche der medizinischen Vorrichtung Titan, Titandioxid oder Zirkoniumdioxid umfasst.

## Revendications

1. Procédé de modification d'une surface d'un dispositif médical implantable dans l'os, comprenant les étapes :
de revêtement d'au moins une première partie de la surface du dispositif médical avec un agent ostéoinducteur pour former une région de surface revêtue ; et
de première irradiation d'au moins une partie de la région de surface revêtue avec un premier faisceau neutre accéléré.

2. Procédé selon la revendication 1, dans lequel l'étape de première irradiation forme une couche superficielle et inférieure peu profonde comprenant des molécules incorporées et/ou des produits de dissociation de l'agent ostéoinducteur.

3. Procédé selon la revendication 1, dans lequel le premier faisceau neutre accéléré est dérivé d'un premier faisceau d'ions d'amas gazeux et en outre dans lequel la couche superficielle et inférieure peu profonde est une couche superficielle imprégnée.

4. Procédé selon la revendication 1, dans lequel l'agent ostéoinducteur comprend, séparément ou en combinaison, l'un quelconque des matériaux du groupe constitué : d'un matériau nutritif, de phosphate tricalcique, d'hydroxyapatite, de bioverre 45S5, de bioverre 58S, d'un agent stimulant la croissance osseuse, d'un facteur de croissance, d'une cytokine, d'une protéine TGF-[bêta], d'une BMP, d'une protéine de signalisation ancrée par GPI, d'une RGM, et d'une protéine régulatrice de croissance.

5. Procédé selon la revendication 1, dans lequel la surface du dispositif médical implantable dans l'os comprend, séparément ou en combinaison, l'un quelconque d'un métal, d'un oxyde ou d'une céramique et, éventuellement, dans lequel la surface comprend du titane, du dioxyde de titane ou de la zircone.

6. Procédé selon la revendication 1, comprenant en outre les étapes, avant l'étape de revêtement, soit :
(a) de formation d'un second faisceau d'ions qui est un faisceau d'ions d'amas gazeux ; et de deuxième irradiation d'au moins une seconde partie de la surface du dispositif médical pour nettoyer l'au moins une seconde partie de la surface ; soit
(b) de formation d'un deuxième faisceau neutre accéléré ; et de deuxième irradiation d'au moins une seconde partie de la surface du dispositif médical pour nettoyer l'au moins une seconde partie de la surface.

7. Procédé de la revendication 1, dans lequel l'étape de première irradiation comprend en outre
(a) l'utilisation d'un masque pour commander l'au moins une partie de la région de surface revêtue qui est irradiée ; ou
(b) le positionnement du dispositif médical par rapport au premier faisceau neutre accéléré pour commander l'au moins une partie de la région de surface revêtue qui est irradiée.

8. Procédé selon la revendication 1, comprenant en outre les étapes :
de formation d'un ou de plusieurs trous dans la surface du dispositif médical ;
de premier chargement d'au moins l'un des un ou plusieurs trous avec un premier agent thérapeutique ; et
de troisième irradiation d'une surface exposée du premier agent thérapeutique dans au moins un trou chargé avec un troisième faisceau neutre accéléré pour former une couche barrière au niveau de la surface exposée.

9. Procédé selon la revendication 8, dans lequel le troisième faisceau neutre accéléré est dérivé d'un faisceau d'ions d'amas gazeux accéléré.

10. Procédé selon la revendication 8, dans lequel la couche barrière commande
(a) une vitesse d'élution d'agent thérapeutique ; ou
(b) une vitesse de diffusion vers l'intérieur d'un fluide dans le trou.

11. Procédé selon la revendication 8, dans lequel les un ou plusieurs trous sont disposés sur la surface selon un motif prédéterminé pour distribuer le premier agent thérapeutique sur la surface selon un plan de distribution prédéterminé.

12. Procédé selon la revendication 8, dans lequel au moins l'un des un ou plusieurs trous est chargé avec
(a) un second agent thérapeutique différent du premier agent thérapeutique ; ou
(b) une première quantité du premier agent thérapeutique qui diffère d'une seconde quantité du premier agent thérapeutique chargé dans au moins un autre des un ou plusieurs trous.

13. Procédé selon la revendication 8, dans lequel l'étape de premier chargement ne remplit pas complètement l'au moins un trou, et, à la suite de l'étape de première irradiation, comprenant en outre les étapes :
de second chargement de l'au moins un trou rempli de manière incomplète avec un second agent thérapeutique recouvrant la première couche barrière ; et
de quatrième irradiation d'une surface exposée du second agent thérapeutique dans au moins un second trou chargé avec un quatrième faisceau neutre accéléré pour former une seconde couche barrière au niveau de la surface exposée dans l'au moins un trou chargé ; et, éventuellement,
dans lequel la première couche barrière et la seconde couche barrière présentent des propriétés différentes pour commander la vitesse d'élution des premier et second agents thérapeutiques.

14. Procédé selon la revendication 13, dans lequel le troisième faisceau neutre accéléré est dérivé d'un faisceau d'ions d'amas gazeux accéléré et en outre, éventuellement, dans lequel le quatrième faisceau neutre accéléré est dérivé d'un faisceau d'ions d'amas gazeux accéléré.

15. Dispositif médical implantable dans l'os présentant une surface, dans lequel au moins une partie de la surface comprend une couche superficielle et inférieure peu profonde imprégnée par faisceau neutre de 10 nanomètres ou moins, comprenant des molécules et/ou des atomes ou des fragments plus grands de molécules d'un agent ostéoinducteur intégré dans l'au moins une partie de la surface.

16. Dispositif médical selon la revendication 15, comprenant en outre :
un ou plusieurs trous contenant un agent thérapeutique à la surface du dispositif médical ; et
au moins une ou plusieurs couches barrières au niveau d'une ou de plusieurs surfaces de l'agent thérapeutique contenu dans les un ou plusieurs trous ; dans lequel
les au moins une ou plusieurs couches barrières commandent au moins une vitesse d'élution d'agent thérapeutique.

17. Dispositif médical selon la revendication 15, dans lequel la surface du dispositif médical comprend du titane, du dioxyde de titane ou de la zircone.
